(19) Europäisches Patentamt
European Patent Office
Office européen des brevets

(11) EP 3 636 767 A1

(12) EUROPEAN PATENT APPLICATION
published in accordance with Art. 153(4) EPC

(43) Date of publication:
**15.04.2020 Bulletin 2020/16**

(21) Application number: **18808936.1**

(22) Date of filing: **28.05.2018**

(51) Int Cl.:
***C12Q 1/26*** *(2006.01)* ***C12M 1/34*** *(2006.01)*
***C12Q 1/28*** *(2006.01)* ***C12Q 1/30*** *(2006.01)*
***G01N 33/72*** *(2006.01)*

(86) International application number:
**PCT/JP2018/020319**

(87) International publication number:
**WO 2018/221446 (06.12.2018 Gazette 2018/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.05.2017 JP 2017107002**

(71) Applicant: **Hitachi Chemical Diagnostics Systems Co., Ltd.**
**Chuo-ku**
**Tokyo 104-6004 (JP)**

(72) Inventors:
- **SOYA, Haruyo**
**Sunto-gun**
**Shizuoka 411-0932 (JP)**
- **HAYASHI, Tatsuya**
**Sunto-gun**
**Shizuoka 411-0932 (JP)**
- **OGAWA, Tomomi**
**Sunto-gun**
**Shizuoka 411-0932 (JP)**
- **KATAYAMA, Yuki**
**Sunto-gun**
**Shizuoka 411-0932 (JP)**

(74) Representative: **Vossius & Partner**
**Patentanwälte Rechtsanwälte mbB**
**Siebertstrasse 3**
**81675 München (DE)**

# (54) METHOD FOR MEASURING GLYCATED HEMOGLOBIN

(57) A method is provided for measuring glycated hemoglobin in a hemoglobin-containing sample which comprises: adding an enzyme that catalyzes a reaction of oxidizing glycated amino acid or glycated peptide to generate hydrogen peroxide without oxidizing the glycated hemoglobin, to the hemoglobin-containing sample to generate hydrogen peroxide; eliminating the generated hydrogen peroxide; adding an enzyme that catalyzes a reaction of oxidizing glycated hemoglobin to generate hydrogen peroxide thereto to generate hydrogen peroxide; and measuring the generated hydrogen peroxide.

EP 3 636 767 A1

## Description

Technical Field

The present invention relates to a method for measuring glycated hemoglobin in a hemoglobin-containing sample, and a measuring kit.

Priority is claimed on Japanese Patent Application No. 2017-107002, filed on May 30, 2017, the content of which is incorporated herein by reference.

Background Art

A glycated protein is contained in biological samples such as a body fluid and hair, and body fluid includes blood in a living body, and such. A concentration of a glycated protein present in blood depends on a concentration of saccharides such as glucose dissolved in serum. In the field of clinical diagnosis, measurement of the concentration of hemoglobin A1c (hereinafter referred to as HbA1c), which is a glycated protein in blood, is used for diagnosis and monitoring of diabetes (see Non-Patent Document 1). Hemoglobin is a hemoprotein consisting of two of each of two types of subunits, the α chain and the β chain, and has a molecular weight of 64,000. HbAlc is defined as hemoglobin in which, in particular, an N-terminal valine residue of β chain is glycated. As a method for measuring this HbA1c, an instrumental analytical method using high-performance liquid chromatography (HPLC) (see Non-Patent Document 2), an immunoassay method using an antigen-antibody reaction (Non-Patent Document 3), and the like are known.

In recent years, development of an enzymatic measurement method for HbA1c, which is applicable to an automatic analyzer with versatility and is also easy to operate, has been in progress, and various methods are being reported. As the enzymatic measurement method for HbA1c, not only a method using protease and fructosyl peptide oxidase, but also a method for measuring glycated hemoglobin in a sample by using an enzyme that catalyzes a reaction of oxidizing glycated hemoglobin to generate hydrogen peroxide without using a protease has been reported (see Patent Documents 1 to 3).

In this enzymatic measurement method for HbA1c, glycated amino acid and glycated peptide in a sample often affect the measurement. In particular, a sample derived from a patient receiving an infusion contains a large amount of glycated amino acid or glycated peptide generated by a reaction of glucose with amino acid or peptide contained in the infusion, which affects the measurement of HbA1c. Until now, methods for measuring glycated hemoglobin in a sample that suppress an effect of glycated amino acid and glycated peptide have been reported (see Patent Documents 4 and 5).

Prior Art Documents

Patent Documents

[Patent Document 1] PCT International Publication No. WO2015/005258
[Patent Document 2] PCT International Publication No. WO2015/005257
[Patent Document 3] PCT International Publication No. WO2015/060429
[Patent Document 4] Japanese Unexamined Patent Application, First Publication No. 2011-045389
[Patent Document 5] PCT International Publication No. WO2003/033729 Non-Patent Documents

[Non-Patent Document 1] Clin Chem Lab Med, Vol. 36, pp. 299 to 308 (1998).
[Non-Patent Document 2] Diabetes, Vol. 27, No. 2, pp. 102 to 107 (1978).
[Non-Patent Document 3] Japanese Journal of Clinical Laboratory Automation, Vol. 18, No. 4, p. 620 (1993).

Summary of Invention

Problems to be Solved by the Invention

Methods for measuring glycated hemoglobin in a hemoglobin-containing sample in the related art, which use an enzyme that catalyzes a reaction of oxidizing glycated hemoglobin to generate hydrogen peroxide without using a protease, have a problem in that glycated hemoglobin could not be measured accurately due to the influence of glycated amino acid or glycated peptide in the hemoglobin-containing sample.

Accordingly, an object of the present invention is to provide a method and a kit for accurately measuring glycated hemoglobin without being affected by glycated amino acid or glycated peptide in a hemoglobin-containing sample.

Means for Solving the Problem

The present inventors have conducted intensive studies to solve such a problem, and have found that glycated hemoglobin can be accurately measured without being affected by glycated amino acid or glycated peptide in a hemoglobin-containing sample, by adding an enzyme that catalyzes a reaction of oxidizing glycated amino acid or glycated peptide to generate hydrogen peroxide without oxidizing the glycated hemoglobin, to the hemoglobin-containing sample to generate hydrogen peroxide; eliminating the generated hydrogen peroxide; adding an enzyme that catalyzes a reaction of oxidizing glycated hemoglobin to generate hydrogen peroxide thereto to generate hydrogen peroxide; and measuring the generated hydrogen peroxide, thus completing the present invention.

That is, the present invention relates to the following [1] to [17].

[1] A method for measuring glycated hemoglobin in a hemoglobin-containing sample, comprising:

adding an enzyme that catalyzes a reaction of oxidizing glycated amino acid or glycated peptide to generate hydrogen peroxide without oxidizing the glycated hemoglobin, to the hemoglobin-containing sample to generate hydrogen peroxide;
eliminating the generated hydrogen peroxide;
adding an enzyme that catalyzes a reaction of oxidizing glycated hemoglobin to generate hydrogen peroxide thereto to generate hydrogen peroxide; and
measuring the generated hydrogen peroxide.

[2] The method according to [1], comprising:

(1) a step of reacting an enzyme that catalyzes a reaction of oxidizing glycated amino acid or glycated peptide to generate hydrogen peroxide without oxidizing glycated hemoglobin with glycated amino acid or glycated peptide in the hemoglobin-containing sample to generate hydrogen peroxide, and eliminating the generated hydrogen peroxide;
(2) a step of adding an enzyme that catalyzes a reaction of oxidizing glycated hemoglobin to generate hydrogen peroxide to a reaction solution in step (1), and reacting the enzyme that catalyzes a reaction of oxidizing glycated hemoglobin to generate hydrogen peroxide with glycated hemoglobin to generate hydrogen peroxide;
(3) a step of measuring the hydrogen peroxide generated in step (2); and
(4) a step of determining a concentration of the glycated hemoglobin in the hemoglobin-containing sample by collating the measured value of step (3) with a calibration curve showing a relationship between a concentration of glycated hemoglobin and a measured value of hydrogen peroxide, prepared in advance by performing the same steps as above (1) to (3), using glycated hemoglobin with known concentrations instead of the hemoglobin-containing sample.

[3] The method according to [1] or [2],
wherein the elimination of the hydrogen peroxide is carried out by catalase.
[4] The method according to [3],
wherein the measurement of the hydrogen peroxide is carried out in the presence of a catalase inhibitor.
[5] The method according to [4],
wherein the catalase inhibitor is azide.
[6] The method according to [1] or [2],
wherein the elimination of the hydrogen peroxide is carried out by a combination of a peroxidase and one of two oxidative coupling-type chromogens used in a chromogenic oxidative coupling reaction.
[7] The method according to any one of [1] to [6],
wherein the elimination of the hydrogen peroxide is carried out by a reducing substance in the sample.
[8] The method according to any one of [1] to [7],
wherein the measurement of the hydrogen peroxide is carried out with a peroxidase and a leuco-type chromogen.
[9] The method according to [8],
wherein the leuco-type chromogen is a phenothiazine-based chromogen.
[10] The method according to [9],
wherin the phenothiazine-based chromogen is 10-(carboxymethylaminocarbonyl)-3,7-bis(dimethylamino)phenothiazine or a salt thereof.

[11] A kit for measuring glycated hemoglobin in a hemoglobin-containing sample, comprising:

a first reagent which comprises catalase and an enzyme that catalyzes a reaction of oxidizing glycated amino acid or glycated peptide to generate hydrogen peroxide without oxidizing glycated hemoglobin; and
a second reagent which comprises a catalase inhibitor and an enzyme that catalyzes a reaction of oxidizing glycated hemoglobin to generate hydrogen peroxide.

[12] The kit according to [11],
wherein the catalase inhibitor is azide.
[13] The kit according to [11] or [12],
wherein a peroxidase and a leuco-type chromogen are further contained in the first reagent and the second reagent, respectively, or in the second reagent and the first reagent, respectively.
[14] A kit for measuring glycated hemoglobin in a hemoglobin-containing sample, comprising:

a first reagent which comprises an enzyme that catalyzes a reaction of oxidizing glycated amino acid or glycated peptide to generate hydrogen peroxide without oxidizing glycated hemoglobin and a combination of a peroxidase and one of two oxidative coupling-type chromogens used in a chromogenic oxidative coupling reaction; and
a second reagent which comprises a leuco-type chromogen and an enzyme that catalyzes a reaction of oxidizing glycated hemoglobin to generate hydrogen peroxide.

[15] A kit for measuring glycated hemoglobin in a hemoglobin-containing sample, comprising:

a first reagent which comprises an enzyme that catalyzes a reaction of oxidizing glycated amino acid or glycated peptide to generate hydrogen peroxide without oxidizing glycated hemoglobin; and
a second reagent which comprises an enzyme that catalyzes a reaction of oxidizing glycated hemoglobin to generate hydrogen peroxide,
wherein a peroxidase and a leuco-type chromogen are contained in the first reagent and the second reagent, respectively, or in the second reagent and the first reagent, respectively.

[16] The kit according to any one of [13] to [15],
wherein the leuco-type chromogen is a phenothiazine-based chromogen.
[17] The kit according to [16],
wherein the phenothiazine-based chromogen is 10-(carboxymethylaminocarbonyl)-3,7-bis(dimethylamino)phenothiazine or a salt thereof.

Effects of the Invention

According to the present invention, a method and a kit are provided for accurately measuring glycated hemoglobin without being affected by glycated amino acid or glycated peptide in a hemoglobin-containing sample.

Brief Description of Drawings

FIG. 1 is a graph showing a relationship between an HbA1c concentration and an absorbance difference $\Delta E$ in measurement of HbAlc in a hemoglobin-containing sample using a kit A containing FPOX-47$\Delta$3 in a second reagent. The vertical axis represents the absorbance difference $\Delta E$ (Abs $\times$ 10,000), and the horizontal axis represents the HbAlc concentration ($\mu$mol/L).

Mode for Carrying out the Invention

1. Method for measuring glycated hemoglobin in hemoglobin-containing sample

The method for measuring glycated hemoglobin in a hemoglobin-containing sample of the present invention is a method characterized by: adding an enzyme that catalyzes a reaction of oxidizing glycated amino acid or glycated peptide to generate hydrogen peroxide without oxidizing the glycated hemoglobin, to the hemoglobin-containing sample to generate hydrogen peroxide; eliminating the generated hydrogen peroxide; adding an enzyme that catalyzes a reaction of oxidizing glycated hemoglobin to generate hydrogen peroxide thereto to generate hydrogen peroxide; and measuring the generated hydrogen peroxide.
Specifically, the method is a method including the following steps.

(1) A step of reacting an enzyme that catalyzes a reaction of oxidizing glycated amino acid or glycated peptide to generate hydrogen peroxide without oxidizing glycated hemoglobin with glycated amino acid or glycated peptide in the hemoglobin-containing sample to generate hydrogen peroxide, and eliminating the generated hydrogen peroxide;
(2) a step of adding an enzyme that catalyzes a reaction of oxidizing glycated hemoglobin to generate hydrogen peroxide to a reaction solution in step (1), and reacting the enzyme that catalyzes a reaction of oxidizing glycated hemoglobin to generate hydrogen peroxide with glycated hemoglobin to generate hydrogen peroxide;
(3) a step of measuring the hydrogen peroxide generated in step (2); and
(4) a step of determining a concentration of the glycated hemoglobin in the hemoglobin-containing sample by collating the measured value of step (3) with a calibration curve showing a relationship between a concentration of glycated hemoglobin and a measured value of hydrogen peroxide, prepared in advance by performing the same steps as above (1) to (3), using glycated hemoglobin with known concentrations instead of the hemoglobin-containing sample.

In addition, the method for measuring glycated hemoglobin in a hemoglobin-containing sample of the present invention also includes a method of calculating a proportion of a concentration of the glycated hemoglobin to a total hemoglobin concentration (that is, a total concentration obtained by combining a concentration of hemoglobin and a concentration of glycated hemoglobin) in the hemoglobin-containing sample. In this case, the method for measuring glycated hemoglobin in a hemoglobin-containing sample of the present invention is, specifically, a measuring method including the following steps.

(1) A step of measuring a total hemoglobin concentration in a hemoglobin-containing sample;
(2) a step of reacting an enzyme that catalyzes a reaction of oxidizing glycated amino acid or glycated peptide to generate hydrogen peroxide without oxidizing glycated hemoglobin with glycated amino acid or glycated peptide in the hemoglobin-containing sample to generate hydrogen peroxide, and eliminating the generated hydrogen peroxide;
(3) a step of adding an enzyme that catalyzes a reaction of oxidizing glycated hemoglobin to generate hydrogen peroxide to a reaction solution in step (2), and reacting the enzyme that catalyzes a reaction of oxidizing glycated hemoglobin to generate hydrogen peroxide with glycated hemoglobin to generate hydrogen peroxide;
(4) a step of measuring the hydrogen peroxide generated in step (3);
(5) a step of determining a concentration of the glycated hemoglobin in the hemoglobin-containing sample by collating the measured value of step (4) with a calibration curve showing a relationship between a concentration of glycated hemoglobin and a measured value of hydrogen peroxide, prepared in advance by performing the same steps as above (2) to (4) using glycated hemoglobin with known concentrations instead of the hemoglobin-containing sample; and
(6) a step of calculating a proportion of a concentration of the glycated hemoglobin to a total hemoglobin concentration in the hemoglobin-containing sample, based on the total hemoglobin concentration measured in step (1) and the concentration of the glycated hemoglobin determined in step (5).

The total hemoglobin concentration can be measured by a known method such as a cyanmethemoglobin method, an oxyhemoglobin method, or an SLS-hemoglobin method.

The hemoglobin-containing sample in the measuring method of the present invention is not particularly limited as long as the hemoglobin-containing sample is a sample which contains hemoglobin and to which the method for measuring glycated hemoglobin of the present invention can be applied. Examples thereof include whole blood, blood cells, a sample in which plasma is mixed in whole blood, and samples obtained by subjecting these samples to hemolysis treatment. The hemolysis treatment is not particularly limited as long as the hemolysis treatment is a treatment for hemolyzing whole blood, blood cells, or a sample in which plasma is mixed in blood cells. Examples thereof include a physical method, a chemical method, and a biological method. Examples of the physical method include a method using a hypotonic solution such as distilled water and a method using ultrasonic waves. Examples of the chemical method include a method using an organic solvent such as methanol, ethanol, and acetone, and a method using a polyoxyethylene-based surfactant. Examples of the biological method include a method using an antibody or complement.

The glycated hemoglobin in the present invention is formed by binding of saccharides such as glucose to hemoglobin, and includes hemoglobin Ala, hemoglobin A1b, HbA1c, and the like, with HbAlc being preferred.

The glycated amino acid in the present invention is present in the hemoglobin-containing sample and can affect the measurement of glycated hemoglobin using an enzyme that catalyzes a reaction of oxidizing glycated hemoglobin to generate hydrogen peroxide. Examples thereof include glycated valine and glycated lysine. Examples of the glycated valine include fructosyl valine. Examples of the glycated lysine include fructosyl lysine.

The glycated peptide in the present invention is present in the hemoglobin-containing sample and can affect the measurement of glycated hemoglobin using an enzyme that catalyzes a reaction of oxidizing glycated hemoglobin to generate hydrogen peroxide. Examples thereof include glycated dipeptide and glycated tripeptide. Examples of the glycated dipeptide include glycated valylhistidine. Examples of the glycated valylhistidine include fructosyl valylhistidine.

In addition, examples of the glycated tripeptide include glycated valylhistidylleucine. Examples of the glycated valylhistidylleucine include fructosyl valylhistidylleucine.

The enzyme that catalyzes a reaction of oxidizing glycated amino acid or glycated peptide to generate hydrogen peroxide without oxidizing glycated hemoglobin is not particularly limited as long as the enzyme is an enzyme that does not act on glycated hemoglobin in a hemoglobin-containing sample but acts on glycated amino acid or glycated peptide in the hemoglobin-containing sample to catalyze a reaction of generating hydrogen peroxide from the glycated amino acid or glycated peptide. Examples thereof include a fructosyl peptide oxidative enzyme derived from filamentous fungi, yeast, actinomycetes, bacteria, and archaea. In the present invention, a commercially available fructosyl peptide oxidative enzyme can also be used. Examples of commercially available products include FPOX-CE (manufactured by Kikkoman Biochemifa Company), FPOX-EE (manufactured by Kikkoman Biochemifa Company), and FPOX-CET (manufactured by Kikkoman Biochemifa Company).

The reaction of the glycated amino acid or glycated peptide in the hemoglobin-containing sample with the enzyme that catalyzes a reaction of oxidizing the glycated amino acid or glycated peptide to generate hydrogen peroxide without oxidizing glycated hemoglobin is preferably carried out in an aqueous medium. The aqueous medium is not particularly limited as long as the aqueous medium enables the method for measuring glycated hemoglobin of the present invention, and examples thereof include deionized water, distilled water, and a buffer solution, with a buffer solution being preferred.

A pH of the aqueous medium is not particularly limited as long as the pH is a pH enables the method for measuring glycated hemoglobin of the present invention, and is, for example, pH 4 to 10. In a case where the buffer solution is used as the aqueous medium, it is desirable to use a buffer according to a pH to be set. Examples of the buffer used for the buffer solution include a tris(hydroxymethyl)aminomethane buffer, a phosphate buffer, a borate buffer, and a Good's buffer.

Examples of the Good's buffer include 2-morpholinoethane sulfonic acid (MES), bis(2-hydroxyethyl)iminotris(hydroxymethyl)methane (Bis-Tris), N-(2-acetamido)iminodiacetic acid (ADA), piperazine-N,N'-bis(2-ethane sulfonic acid) (PIPES), N-(2-acetamido)-2-aminoethane sulfonic acid (ACES), 3-morpholino-2-hydroxypropane sulfonic acid (MOPSO), N,N-bis(2-hydroxyethyl)-2-aminoethane sulfonic acid (BES), 3-morpholinopropane sulfonic acid (MOPS), N-[tris(hydroxymethyl)methyl]-2-aminoethane sulfonic acid (TES), 2-[4-(2-hydroxyethyl)-1-piperazinyl]ethane sulfonic acid (HEPES), 3-[N,N-bis(2-hydroxyethyl)amino]-2-hydroxypropane sulfonic acid (DIPSO), N-[tris(hydroxymethyl)methyl]-2-hydroxy-3-aminopropane sulfonic acid (TAPSO), piperazine-N,N'-bis(2-hydroxypropanesulfonic acid) (POPSO), 3-[4-(2-hydroxyethyl)-1-piperazinyl]-2-hydroxypropane sulfonic acid (HEPPSO), 3-[4-(2-hydroxyethyl)-1-piperazinyl]propane sulfonic acid [(H)EPPS], N-[tris(hydroxymethyl)methyl]glycine (Tricine), N,N-bis(2-hydroxyethyl)glycine (Bicine), N-tris(hydroxymethyl)methyl-3-aminopropane sulfonic acid (TAPS), N-cyclohexyl-2-aminoethane sulfonic acid (CHES), N-cyclohexyl-3-amino-2-hydroxypropane sulfonic acid (CAPSO), and N-cyclohexyl-3-aminopropane sulfonic acid (CAPS). A concentration of the buffer solution is usually 0.001 to 2.0 mol/L, and preferably 0.005 to 1.0 mol/L.

In the reaction of the glycated amino acid or glycated peptide in the hemoglobin-containing sample with the enzyme that catalyzes a reaction of oxidizing the glycated amino acid or glycated peptide to generate hydrogen peroxide without oxidizing glycated hemoglobin, a reaction temperature is not particularly limited as long as the reaction temperature enables the reaction, and is usually 10°C to 50°C, and preferably 20°C to 40°C. In addition, a reaction time in the reaction is not particularly limited as long as the reaction time enables the reaction, and is usually 1 minute to 3 hours, and preferably 2.5 minutes to 1 hour. A concentration of the enzyme that catalyzes a reaction of oxidizing glycated amino acid or glycated peptide to generate hydrogen peroxide without oxidizing glycated hemoglobin in a reaction solution is not particularly limited as long as the concentration enables to proceed the reaction of the glycated amino acid or glycated peptide in the hemoglobin-containing sample with the enzyme that catalyzes the reaction of oxidizing glycated amino acid or glycated peptide to generate hydrogen peroxide without oxidizing glycated hemoglobin, and is usually 0.1 to 30 kU/L, and preferably 0.2 to 15 kU/L.

In the present invention, eliminating hydrogen peroxide generated in the reaction of glycated amino acid or glycated peptide in the hemoglobin-containing sample with the enzyme that catalyzes a reaction of oxidizing glycated amino acid or glycated peptide to generate hydrogen peroxide without oxidizing glycated hemoglobin means that hydrogen peroxide generated in a reaction of glycated amino acid or glycated peptide in the hemoglobin-containing sample with the enzyme that catalyzes a reaction of oxidizing glycated amino acid or glycated peptide to generate hydrogen peroxide without oxidizing glycated hemoglobin is converted into a substance that does not affect the measurement of glycated hemoglobin in the hemoglobin-containing sample. Eliminating hydrogen peroxide generated in the reaction of glycated amino acid or glycated peptide in the hemoglobin-containing sample with the enzyme that catalyzes a reaction of oxidizing glycated amino acid or glycated peptide to generate hydrogen peroxide without oxidizing glycated hemoglobin can be carried out by, for example, catalase, a combination of a peroxidase and one of two oxidative coupling-type chromogens used in a chromogenic oxidative coupling reaction or a reducing substance in the hemoglobin-containing sample. Examples of the oxidative coupling-type chromogen include an oxidative coupling-type chromogen to be described later. Examples of the reducing substance in the hemoglobin-containing sample include ascorbic acid and hemoglobin.

In a case where the hydrogen peroxide generated in the reaction of glycated amino acid or glycated peptide in the hemoglobin-containing sample with the enzyme that catalyzes a reaction of oxidizing glycated amino acid or glycated peptide to generate hydrogen peroxide without oxidizing glycated hemoglobin is eliminated by the catalase, a concentration of the catalase in the reaction solution is not particularly limited as long as the concentration thereof enables the method for measuring glycated hemoglobin of the present invention, and is usually 0.001 to 5,000 kU/L and preferably 0.01 to 1,000 kU/L.

In a case where the hydrogen peroxide generated in the reaction of glycated amino acid or glycated peptide in the hemoglobin-containing sample with the enzyme that catalyzes a reaction of oxidizing glycated amino acid or glycated peptide to generate hydrogen peroxide without oxidizing glycated hemoglobin is eliminated by a combination of a peroxidase and one of two oxidative coupling-type chromogens used in a chromogenic oxidative coupling reaction, a concentration of the peroxidase in a reaction solution is not particularly limited as long as the concentration thereof enables the method for measuring glycated hemoglobin of the present invention, and is usually 0.01 to 500 kU/L and preferably 0.1 to 100 kU/L. A concentration of the one of two oxidative coupling-type chromogens used in a chromogenic oxidative coupling reaction in a reaction solution is not particularly limited as long as the concentration thereof enables the method for measuring glycated hemoglobin of the present invention, and is usually 0.01 to 10 g/L and preferably 0.05 to 5 g/L.

In the reaction of the glycated hemoglobin in the hemoglobin-containing sample with the enzyme that catalyzes a reaction of oxidizing the glycated hemoglobin to generate hydrogen peroxide, a reaction temperature is not particularly limited as long as the reaction temperature enables the method for measuring glycated hemoglobin of the present invention, and is usually 10°C to 50°C, and preferably 20°C to 40°C. In addition, a reaction time in the reaction is not particularly limited as long as the reaction time enables the method for measuring glycated hemoglobin of the present invention, and is usually 1 minute to 3 hours, and preferably 2.5 minutes to 1 hour. A concentration of the enzyme that catalyzes a reaction of oxidizing glycated hemoglobin to generate hydrogen peroxide in a reaction solution is not particularly limited as long as the concentration enables the method for measuring glycated hemoglobin of the present invention, and is usually 0.1 to 30 kU/L, and preferably 0.2 to 15 kU/L.

In addition, the reaction of the glycated hemoglobin in the hemoglobin-containing sample with the enzyme that catalyzes a reaction of oxidizing the glycated hemoglobin to generate hydrogen peroxide is preferably carried out in the presence of a glycated hemoglobin denaturing agent that changes the structure of the glycated hemoglobin in the hemoglobin-containing sample to enable a reaction with the enzyme that catalyzes a reaction of oxidizing the glycated hemoglobin to generate hydrogen peroxide. Examples of the glycated hemoglobin denaturing agent include a nonionic surfactant, a cationic surfactant, an anionic surfactant, and an amphoteric surfactant. Examples of the nonionic surfactant include alkyl glucoside, fatty acid sorbitan ester, fatty acid diethanolamide, polyoxyethylene alkyl ether, and alkyl monoglyceryl ether. Examples of the cationic surfactant include a quaternary ammonium salt, a pyridinium salt, a phosphonium salt, an imidazolium salt, and an isoquinonium salt. Examples of the anionic surfactant include N-acyl taurine, alkyl sulfoacetic acid, polyoxyethylene alkyl ether acetic acid, N-acyl amino acid, polyoxyethylene alkyl ether phosphoric acid, polyoxyethylene polycyclic phenyl ether phosphoric acid, alkyl phosphoric acid, a linear alkylbenzene sulfonic acid salt, an alkyl sulfonic acid salt, and a polyoxyethylene alkyl ether sulfonic acid salt. Examples of the amphoteric surfactant include alkyl carboxybetaine and alkyldimethylamine oxide.

In a case where the hydrogen peroxide generated in the reaction of glycated amino acid or glycated peptide in the hemoglobin-containing sample with the enzyme that catalyzes a reaction of oxidizing glycated amino acid or glycated peptide to generate hydrogen peroxide without oxidizing glycated hemoglobin is eliminated by a catalase, the reaction of glycated hemoglobin in the hemoglobin-containing sample with the enzyme that catalyzes the reaction of oxidizing glycated hemoglobin to generate hydrogen peroxide is preferably carried out in the presence of a catalase inhibitor. Examples of the catalase inhibitor include azide. Examples of the azide include lithium azide, sodium azide, and potassium azide. A concentration of the catalase inhibitor in the reaction solution is not particularly limited as long as the concentration enables the method for measuring glycated hemoglobin of the present invention, and is usually 0.5 to 60 mmol/L, and preferably 1 to 30 mmol/L.

The measurement of the hydrogen peroxide generated in the reaction of the glycated hemoglobin in the hemoglobin-containing sample with the enzyme that catalyzes the reaction of oxidizing glycated hemoglobin to generate hydrogen peroxide is not particularly limited as long as the measurement enables the method for measuring glycated hemoglobin of the present invention. Examples thereof include measurement using a hydrogen peroxide electrode, measurement using peroxidase and a leuco-type chromogen, measurement using peroxidase and a chemiluminescent substance, and measurement using peroxidase and a fluorescent substance, with the measurement using peroxidase and leuco-type chromogen being preferred.

In addition, in a case where the hydrogen peroxide generated in the reaction of glycated amino acid or glycated peptide in the hemoglobin-containing sample with the enzyme that catalyzes a reaction of oxidizing glycated amino acid or glycated peptide to generate hydrogen peroxide without oxidizing glycated hemoglobin is eliminated by using one of two oxidative coupling-type chromogens used in a chromogenic oxidative coupling reaction, the measurement of the hydrogen peroxide generated in the reaction of the glycated hemoglobin in the hemoglobin-containing sample with the enzyme that catalyzes the reaction of oxidizing glycated hemoglobin to generate hydrogen peroxide can be carried out by using peroxidase and one of two oxidative coupling-type chromogens used in a chromogenic oxidative coupling reaction as well as the other one of the two oxidative coupling-type chromogens used in a chromogenic oxidative coupling reaction.

Examples of the leuco-type chromogen include a leuco-type chromogen to be described later. Examples of the chemiluminescent substance include luminol, isoluminol, lucigenin, and acridinium ester. Examples of the fluorescent substance include 4-hydroxyphenylacetic acid, 3-(4-hydroxyphenyl)propionic acid, and coumarin.

In a case where the measurement of the hydrogen peroxide generated in the reaction of glycated hemoglobin in the hemoglobin-containing sample with the enzyme that catalyzes a reaction of oxidizing glycated hemoglobin to generate hydrogen peroxide is carried out by the peroxidase and the leuco-type chromogen, a concentration of the peroxidase in a reaction solution is not particularly limited as long as the concentration thereof enables the method for measuring glycated hemoglobin of the present invention, and is usually 0.01 to 500 kU/L and preferably 0.1 to 100 kU/L, and a concentration of the leuco-type chromogen in a reaction solution is not particularly limited as long as the concentration thereof enables the method for measuring glycated hemoglobin of the present invention, and is usually 0.001 to 10 g/L and is preferably 0.005 to 5 g/L.

In addition, in a case where the measurement of the hydrogen peroxide generated in the reaction of glycated hemoglobin in the hemoglobin-containing sample with the enzyme that catalyzes a reaction of oxidizing glycated hemoglobin to generate hydrogen peroxide is carried out by the peroxidase and the two oxidative coupling-type chromogens used in a chromogenic oxidative coupling reaction, a concentration of the peroxidase in a reaction solution is not particularly limited as long as the concentration thereof enables the method for measuring glycated hemoglobin of the present invention, and is usually 0.01 to 500 kU/L and preferably 0.1 to 100 kU/L, a concentration of one of the two oxidative coupling-type chromogens (for example, a coupler to be described later) in a reaction solution is not particularly limited as long as the concentration thereof enables the method for measuring glycated hemoglobin of the present invention, and is usually 0.001 to 10 g/L and preferably 0.005 to 5 g/L, and a concentration of the other one of the two oxidative coupling-type chromogens (for example, phenols or anilines to be described later) in a reaction solution is not particularly limited as long as the concentration thereof enables the method for measuring glycated hemoglobin of the present invention, and is usually 0.001 to 10 g/L and preferably 0.005 to 5 g/L.

In the present invention, an enzyme activity (U) of the enzyme that catalyzes a reaction of oxidizing glycated hemoglobin to generate hydrogen peroxide can be calculated, for example, by the following method.

<Reagent for measuring enzyme activity>

First reagent

Phosphate buffer solution (pH 8.0) 0.1 mmol/L
N-Ethyl-N-(3-methylphenyl)-N'-succinyl ethylenediamine (EMSE) 0.3 g/L
Peroxidase 3 kU/L
4-Aminoantipyrine 0.1 g/L

Second reagent

Fructosyl valine aqueous solution 1.7 mmol/L

The fructosyl valine aqueous solution was prepared by the method described in J. Agric. Food Chem., Vol. 24, No. 1, pp.70 to 73 (1976).

<Sample diluent>

Phosphate buffer solution (pH 8.0) 10 mmol/L
Bovine serum albumin 0.15%

<Sample>

FPOX-47Δ3 having an amino acid sequence represented by SEQ ID NO: 10 is diluted with the above-mentioned sample diluent and used.

<Measurement>

The sample (2 μL) and the first reagent (150 μL) of the reagent for measuring an enzyme activity are added to a reaction cuvette and allowed to react at 37°C for 3 minutes (first reaction). Then, the second reagent (20 μL) of the reagent for measuring an enzyme activity is added, and an absorbance [$Abs_{enzyme}$ (after 3 minutes)] of a reaction solution 3 minutes after addition of the second reagent and an absorbance [$Abs_{enzyme}$ (after 5 minutes)] of a reaction solution 5 minutes after addition of the second reagent are both measured at a main wavelength of 546 nm and a sub-wavelength of 700 nm. The $Abs_{enzyme}$ (after 3 minutes) is subtracted from the $Abs_{enzyme}$ (after 5 minutes) to calculate an absorbance difference $\Delta Abs'_{enzyme}$, and the calculated absorbance difference $\Delta Abs'_{enzyme}$ is divided by 2 (minutes) to calculate an absorbance change amount per minute $\Delta Abs'_{enzyme}$/min.

An absorbance difference $\Delta Abs'_{blank}$/min is calculated by the same method except that the sample diluent is used in place of the above sample.

An absorbance difference $\Delta Abs_{enzyme}$/min for the enzyme is determined by subtracting the ΔAbs'blank/min from the calculated $\Delta Abs'_{enzyme}$/min.

<Enzyme activity>

Based on the $\Delta Abs_{enzyme}$/min determined above, an enzyme activity is calculated by the following expression (I).

$$\text{Enzymatic activity (U/mL)} = \frac{\Delta Abs_{enzyme}/\text{min} \times 0.172\ (\text{mL}) \times \text{dilution rate}}{33.8 \times 0.5 \times 0.002\ (\text{mL})} \quad (I)$$

In expression (I), 33.8 is an extinction coefficient in mmol [$(mmol/L)^{-1} \cdot cm^{-1}$] of a quinonimine dye generated by reaction.

In expression (I), 0.5 is the number of mol of a quinonimine dye generated by 1 mol of hydrogen peroxide.

The enzyme that catalyzes a reaction of oxidizing glycated hemoglobin to generate hydrogen peroxide is not particularly limited as long as the enzyme is an enzyme that acts on glycated hemoglobin in a hemoglobin-containing sample and generates hydrogen peroxide from the glycated hemoglobin, and examples thereof include the enzymes described in PCT International Publication No. WO2015/005258, and the amadoriases described in PCT International Publication No. WO2015/060429. Specifically, FPOX-18A, FPOX-18B, FPOX-18C, FPOX-18D, and FPOX-19 to 46 described in PCT International Publication No. WO2015/005258 and modified products of the enzymes can be mentioned. As specific examples of modified products of the enzymes, for example, FPOX-47Δ3 having an amino acid sequence represented by SEQ ID NO: 10 and the like can be mentioned.

In the present invention, the leuco-type chromogen is a substance which is converted into a dye by itself in the presence of hydrogen peroxide and a peroxidative substance. Examples of the peroxidative substance include a peroxidase.

Examples of the leuco-type chromogen include a phenothiazine-based chromogen, a triphenylmethane-based chromogen, a diphenylamine-based chromogen, o-phenylenediamine, hydroxypropionic acid, diaminobenzidine, and tetramethylbenzidine, with a phenothiazine-based chromogen being preferred.

Examples of the phenothiazine-based chromogen include 10-N-carboxymethylcarbamoyl-3,7-bis(dimethylamino)-10H-phenothiazine (CCAP), 10-N-methylcarbamoyl-3,7-bis(dimethylamino)-10H-phenothiazine (MCDP), and 10-N-(carboxymethylaminocarbonyl)-3,7-bis(dimethylamino)-10H-phenothiazine sodium salt (DA-67). Among the phenothiazine-based chromogens, 10-N-(carboxymethylaminocarbonyl)-3,7-bis(dimethylamino)-10H-phenothiazine sodium salt (DA-67) is particularly preferable.

Examples of the triphenylmethane-based chromogen include N,N,N',N',N",N"-hexa(3-sulfopropyl)-4,4',4"-triaminotriphenyl methane (TPM-PS). Examples of the diphenylamine-based chromogen include N-(carboxymethylaminocarbonyl)-4,4'-bis(dimethylamino)diphenylamine sodium salt (DA-64), 4,4'-bis(dimethylamino)diphenylamine, and bis[3-bis(4-chlorophenyl)methyl-4-dimethylaminophenyl]amine (BCMA).

In the present invention, the oxidative coupling-type chromogen is a substance that forms dye by oxidative-coupling two compounds in the presence of hydrogen peroxide and a peroxidative substance. As a combination of the two compounds, a combination of a coupler and aniline, a combination of a coupler and phenol, and the like c mentioned.

Examples of the coupler include 4-aminoantipyrine (4-AA) and 3-methyl-2-benzothiazolinone hydrazine.

Examples of the anilines include N-(3-sulfopropyl)aniline, N-ethyl-N-(2-hydroxy-3-sulfopropyl)-3-methylaniline (TOOS), N-ethyl-N-(2-hydroxy-3-sulfopropyl)-3,5-dimethylaniline (MAOS), N-ethyl-N-(2-hydroxy-3-sulfopropyl)-3,5-dimethoxyaniline (DAOS), N-ethyl-N-(3-sulfopropyl)-3-methylaniline (TOPS), N-(2-hydroxy-3-sulfopropyl)-3,5-dimethoxyaniline (HDAOS), N,N-dimethyl-3-metylaniline, N,N-bis(3-sulfopropyl)-3,5-dimethoxyaniline, N-ethyl-N-(3-sulfopropyl)-3-methoxyaniline, N-ethyl-N-(3-sulfopropyl)aniline, N-ethyl-N-(3-sulfopropyl)-3,5-dimethoxaniline, N-(3-sulfopropyl)-3,5-dimethoxyaniline, N-ethyl-N-(3-sulfopropyl)-3,5-dimethylaniline, N-ethyl-N -(2-hydroxy-3-sulfopropyl)-3-methoxyaniline, N-ethyl-N-(2-hydroxy-3-sulfopropyl)aniline, N-ethyl-N-(3-methylphenyl)-N'-succinyl ethylenediamine (EMSE), N-ethyl-N-(3-methylphenyl)-N'-acetyl ethylenediamine, and N-ethyl-N-(2-hydroxy-3-sulfopropyl)-4-fluoro-3,5-dimethoxyaniline (F-DAOS).

Examples of the phenols include phenol, 4-chlorophenol, 3-methylphenol, and 3-hydroxy-2,4,6-triiodobenzoic acid (HTIB).

2. Kit for measuring glycated hemoglobin in hemoglobin-containing sample

The kit for measuring glycated hemoglobin in a hemoglobin-containing sample of the present invention is used in the method for measuring glycated hemoglobin in a hemoglobin-containing sample of the present invention.

Examples of the measuring kit of the present invention include a two-reagent system kit and a three-reagent system kit, with the two-reagent system kit being preferred.

The kit for measuring glycated hemoglobin in a hemoglobin-containing sample of the present invention is a kit comprising a first reagent which comprises catalase and an enzyme that catalyzes a reaction of oxidizing glycated amino acid or glycated peptide to generate hydrogen peroxide without oxidizing glycated hemoglobin and a second reagent which comprises a catalase inhibitor and an enzyme that catalyzes a reaction of oxidizing glycated hemoglobin to generate hydrogen peroxide, and is used in the method for measuring glycated hemoglobin of the present invention. In the kit, a peroxidase and a leuco-type chromogen may be contained in the first reagent and the second reagent, respectively, or in the second reagent and the first reagent, respectively.

In addition, the kit for measuring glycated hemoglobin in a hemoglobin-containing sample of the present invention is a kit comprising a first reagent which comprises an enzyme that catalyzes a reaction of oxidizing glycated amino acid or glycated peptide to generate hydrogen peroxide without oxidizing glycated hemoglobin and a combination of a peroxidase and one of two oxidative coupling-type chromogens used in a chromogenic oxidative coupling reaction and a second reagent which comprises a leuco-type chromogen and an enzyme that catalyzes a reaction of oxidizing glycated hemoglobin to generate hydrogen peroxide, and is used in the method for measuring glycated hemoglobin of the present invention.

Furthermore, the kit for measuring glycated hemoglobin in a hemoglobin-containing sample of the present invention is a kit comprising a first reagent which comprises an enzyme that catalyzes a reaction of oxidizing glycated amino acid or glycated peptide to generate hydrogen peroxide without oxidizing glycated hemoglobin; and a second reagent which comprises an enzyme that catalyzes a reaction of oxidizing glycated hemoglobin to generate hydrogen peroxide, in which a peroxidase and a leuco-type chromogen are contained in the first reagent and the second reagent, respectively, or in the second reagent and the first reagent, respectively, and is used in the method for measuring glycated hemoglobin of the present invention.

In the kit for measuring glycated hemoglobin of the present invention, examples of an enzyme that catalyzes a reaction of oxidizing glycated amino acid or glycated peptide to generate hydrogen peroxide without oxidizing glycated hemoglobin and an enzyme that catalyzes a reaction of oxidizing glycated hemoglobin to generate hydrogen peroxide include the enzyme that catalyzes a reaction of oxidizing glycated amino acid or glycated peptide to generate hydrogen peroxide without oxidizing the glycated hemoglobin and the enzyme that catalyzes a reaction of oxidizing glycated hemoglobin to generate hydrogen peroxide, respectively, as mentioned above. In the kit for measuring glycated hemoglobin of the present invention, examples of a catalase inhibitor, an oxidative coupling-type chromogen, and a leuco-type chromogen include the catalase inhibitor, the oxidative coupling-type chromogen, and the leuco-type chromogen, respectively, as mentioned above.

In a case where the kit for measuring glycated hemoglobin of the present invention is used to measure glycated hemoglobin in a hemoglobin-containing sample, the glycated hemoglobin can be measured by adding the hemoglobin-containing sample and a first reagent to a reaction cuvette to carry out a reaction at a predetermined temperature for a predetermined time, and then adding a second reagent, and measuring the generated hydrogen peroxide, according to the method for measuring glycated hemoglobin in a hemoglobin-containing sample of the present invention mentioned above.

Specific embodiments of the kit for measuring glycated hemoglobin of the present invention are described below.

- Kit 1

A kit for measuring glycated hemoglobin, containing:

a first reagent which comprises catalase and an enzyme that catalyzes a reaction of oxidizing glycated amino acid or glycated peptide to generate hydrogen peroxide without oxidizing glycated hemoglobin; and
a second reagent which comprises a catalase inhibitor and an enzyme that catalyzes a reaction of oxidizing glycated hemoglobin to generate hydrogen peroxide.

• Kit 2

A kit for measuring glycated hemoglobin, containing:

a first reagent which comprises an enzyme that catalyzes a reaction of oxidizing glycated amino acid or glycated peptide to generate hydrogen peroxide without oxidizing glycated hemoglobin, a peroxidase, and catalase; and
a second reagent which comprises an enzyme that catalyzes a reaction of oxidizing glycated hemoglobin to generate hydrogen peroxide, a catalase inhibitor, and a leuco-type chromogen.

• Kit 3

A kit for measuring glycated hemoglobin, containing:

a first reagent which comprises an enzyme that catalyzes a reaction of oxidizing glycated amino acid or glycated peptide to generate hydrogen peroxide without oxidizing glycated hemoglobin, leuco-type chromogen, and catalase; and
a second reagent which comprises an enzyme that catalyzes a reaction of oxidizing glycated hemoglobin to generate hydrogen peroxide, a catalase inhibitor, and a peroxidase.

• Kit 4

A kit for measuring glycated hemoglobin, containing:

a first reagent which comprises an enzyme that catalyzes a reaction of oxidizing glycated amino acid or glycated peptide to generate hydrogen peroxide without oxidizing glycated hemoglobin, a peroxidase, and one of two oxidative coupling-type chromogens used in a chromogenic oxidative coupling reaction; and
a second reagent which comprises a leuco-type chromogen and an enzyme that catalyzes a reaction of oxidizing glycated hemoglobin to generate hydrogen peroxide.

• Kit 5

A kit for measuring glycated hemoglobin, containing:

a first reagent which comprises an enzyme that catalyzes a reaction of oxidizing glycated amino acid or glycated peptide to generate hydrogen peroxide without oxidizing glycated hemoglobin, a peroxidase, and one of two oxidative coupling-type chromogens used in a chromogenic oxidative coupling reaction; and
a second reagent which comprises an enzyme that catalyzes a reaction of oxidizing glycated hemoglobin to generate hydrogen peroxide and the other one of two oxidative coupling-type chromogens used in a chromogenic oxidative coupling reaction.

• Kit 6

A kit for measuring glycated hemoglobin, containing:

a first reagent which comprises a peroxidase and an enzyme that catalyzes a reaction of oxidizing glycated amino acid or glycated peptide to generate hydrogen peroxide without oxidizing glycated hemoglobin; and
a second reagent which comprises an enzyme that catalyzes a reaction of oxidizing glycated hemoglobin to generate hydrogen peroxide, and a leuco-type chromogen.

• Kit 7

A kit for measuring glycated hemoglobin, containing:

a first reagent which comprises a leuco-type chromogen and an enzyme that catalyzes a reaction of oxidizing glycated amino acid or glycated peptide to generate hydrogen peroxide without oxidizing glycated hemoglobin; and a second reagent which comprises a peroxidase and an enzyme that catalyzes a reaction of oxidizing glycated hemoglobin to generate hydrogen peroxide.

The first reagent and/or the second reagent comprised of the kit for measuring glycated hemoglobin of the present invention may be in a freeze-dried state or in a state of being dissolved in an aqueous medium. Examples of the aqueous medium include the aqueous medium mentioned above. In a case where the first reagent and/or the second reagent in a freeze-dried state is used to measure glycated hemoglobin in a hemoglobin-containing sample, the first reagent and/or the second reagent in a freeze-dried are used by being dissolved in an aqueous medium. Examples of the aqueous medium include the aqueous medium mentioned above.

In the first reagent comprised of the measuring kit of the present invention, a content of the enzyme that catalyzes a reaction of oxidizing glycated amino acid or glycated peptide to generate hydrogen peroxide without oxidizing glycated hemoglobin is usually such a content that a concentration in a state of being dissolved in an aqueous medium can be 0.1 to 60 kU/L, with such a content that the concentration can be 0.2 to 30 kU/L being preferred.

In the second reagent comprised of the measuring kit of the present invention, a content of the enzyme that catalyzes a reaction of oxidizing glycated hemoglobin to generate hydrogen peroxide is usually such a content that a concentration in a state of being dissolved in an aqueous medium can be 0.1 to 120 kU/L, with such a content that the concentration can be 0.2 to 60 kU/L being preferred.

In the first reagent comprised of the measuring kit of the present invention, a content of the catalase is usually such a content that a concentration in a state of being dissolved in an aqueous medium can be 0.002 to 10,000 kU/L, with such a content that the concentration can be 0.02 to 2,000 kU/L being preferred.

In the second reagent comprised of the measuring kit of the present invention, a content of the catalase inhibitor is usually such a content that a concentration in a state of being dissolved in an aqueous medium can be 2 to 240 mmol/L, with such a content that the concentration can be 4 to 120 mmol/L being preferred.

In the first reagent comprised of the measuring kit of the present invention, a content of the peroxidase is usually such a content that a concentration in a state of being dissolved in an aqueous medium can be 0.02 to 1,000 kU/L, with such a content that the concentration can be 0.2 to 200 kU/L being preferred.

In the first reagent comprised of the measuring kit of the present invention, a content of one of two oxidative coupling-type chromogens used in a chromogenic oxidative coupling reaction is usually such a content that a concentration in a state of being dissolved in an aqueous medium can be 0.02 to 20 g/L, with such a content that the concentration can be 0.1 to 10 g//L being preferred.

In the second reagent comprised of the measuring kit of the present invention, a content of the leuco-type chromogen is usually such a content that a concentration in a state of being dissolved in an aqueous medium can be 0.004 to 40 g/L, with such a content that the concentration can be 0.02 to 20 g/L being preferred.

In the second reagent comprised of the measuring kit of the present invention, a content of the peroxidase is usually such a content that a concentration in a state of being dissolved in an aqueous medium can be 0.04 to 2,000 kU/L, with such a content that the concentration can be 0.4 to 400 kU/L being preferred.

In the first reagent comprised of the measuring kit of the present invention, a content of the leuco-type chromogen is usually such a content that a concentration in a state of being dissolved in an aqueous medium can be 0.002 to 20 g/L, with such a content that the concentration can be 0.01 to 10 g/L being preferred.

In the second reagent comprised of the measuring kit of the present invention, a content of the other one of two oxidative coupling-type chromogens used in a chromogenic oxidative coupling reaction is usually such a content that a concentration in a state of being dissolved in an aqueous medium can be 0.04 to 40g/L, with such a content that the concentration can be 0.2 to 20 g//L being preferred.

The kit for measuring glycated hemoglobin of the present invention may contain, as necessary, a stabilizer, an antiseptic, salts, an interference substance elimination agent, an organic solvent, and the like, in addition to the above-mentioned aqueous medium and the above-mentioned glycated hemoglobin denaturing agent. Examples of the stabilizer include ethylenediaminetetraacetic acid (EDTA), sucrose, calcium chloride, calcium acetate, calcium nitrate, potassium ferrocyanide, bovine serum albumin (BSA), and a polyoxyethylene-based surfactant such as polyoxyethylene alkyl phenyl ether. Examples of the antiseptic include sodium azide and antibiotics. Examples of the salt include sodium chloride, sodium nitrate, sodium sulfate, sodium carbonate, sodium formate, sodium acetate, potassium chloride, potassium nitrate, potassium sulfate, potassium carbonate, potassium formate, and potassium acetate. Examples of the interference substance elimination agent include ascorbic acid oxidase for eliminating the influence of ascorbic acid. Examples of the organic solvent include dimethylformamide (DMF), dimethylsulfoxide (DMSO), dioxane, acetone, methanol, and ethanol as dissolution auxiliary agents for a leuco-type chromogen in an aqueous medium. Examples

Hereinafter, the present invention will be described in more detail with reference to examples, but these examples do not limit the scope of the present invention in any way. In the present examples, comparative examples, and experimental examples, reagents and enzymes of the following manufacturers were used.

Bis-Tris (manufactured by Dojindo Laboratories), FPOX-CET (enzyme that catalyzes a reaction of oxidizing glycated amino acid or glycated peptide to generate hydrogen peroxide without oxidizing glycated hemoglobin; manufactured by Kikkoman Biochemifa Company), DA -67 (manufactured by Wako Pure Chemical Industries, Ltd.), 4-aminoantipyrine (manufactured by Saikyo Kasei Co., Ltd.), EMSE (manufactured by Dojindo Laboratories), a peroxidase (manufactured by Toyobo Co., Ltd.), catalase (manufactured by Toyobo Co., Ltd), NIKKOL LMT [N-acyl taurine salt (sodium N-lauroyl-N-methyl taurine); manufactured by Nikko Chemicals Co., Ltd.], sodium azide (manufactured by Wako Pure Chemical Industries, Ltd.), fructosyl valine (prepared by the method described in J. Agric. Food Chem., Vol. 24, No. 1, pp.70 to 73 (1976)).

FPOX-47Δ3, which is an enzyme that catalyzes a reaction of oxidizing glycated hemoglobin to generate hydrogen peroxide, was prepared by the method of Reference Example 1 to be described later. In addition, an enzyme activity of FPOX-47Δ3 was calculated based on the above-mentioned expression (I).

[Example 1]

Kits for measuring HbAlc comprising the following first reagent and second reagent were prepared.

First reagent

| | |
|---|---|
| Bis-Tris (pH 7.0) | 50 mmol/L |
| NIKKOL LMT | 1.5 g/L |
| FPOX-CET | 1 kU/L |
| DA-67 | 40 μmol/L |
| Catalase | 100 kU/L |

Second reagent

| | |
|---|---|
| Bis-Tris (pH 7.0) | 50 mmol/L |
| FPOX-47Δ3 | 1.5 kU/L |
| Peroxidase | 200 kU/L |
| Sodium azide | 0.1 g/L |

[Example 2]

Kits for measuring HbA1c comprising the following first reagent and second reagent were prepared.

First reagent

| | |
|---|---|
| Bis-Tris (pH 7.0) | 50 mmol/L |
| NIKKOL LMT | 1.5 g/L |
| FPOX-CET | 1kU/L |
| Peroxidase 40 | kU/L |
| Catalase | 100kU/L |

Second reagent

| | |
|---|---|
| Bis-Tris (pH 7.0) | 50 mmol/L |
| FPOX-47Δ3 | 1.5 kU/L |
| DA-67 | 120 μmol/L |
| Sodium azide | 0.1 g/L |

[Example 3]

Kits for measuring HbAlc comprising the following first reagent and second reagent were prepared. This kit is the same as the kit of Example 1 except that catalase is not contained in the first reagent.

| First reagent | |
|---|---|
| Bis-Tris (pH 7.0) | 50 mmol/L |
| NIKKOL LMT | 1.5 g/L |
| FPOX-CET | 1kU/L |
| DA-67 | 40 μmol/L |

| Second reagent | |
|---|---|
| Bis-Tris (pH 7.0) | 50 mmol/L |
| FPOX-47Δ3 | 1.5 kU/L |
| Peroxidase | 200 kU/L |
| Sodium azide | 0.1 g/L |

[Example 4]

Kits for measuring HbAlc comprising the following first reagent and second reagent were prepared. This kit is the same as the kit of Example 2 except that catalase is not contained in the first reagent.

| First reagent | |
|---|---|
| Bis-Tris (pH 7.0) | 50 mmol/L |
| NIKKOL LMT | 1.5 g/L |
| FPOX-CET | 1kU/L |
| Peroxidase | 40kU/L |

| Second reagent | |
|---|---|
| Bis-Tris (pH 7.0) | 50 mmol/L |
| FPOX-47Δ3 | 1.5 kU/L |
| DA-67 | 120 μmol/L |
| Sodium azide | 0.1 g/L |

[Example 5]

Using the kit of Example 1 as a kit for measuring HbA1c, and using 5 concentrations of whole blood in which HbAlc concentrations were value-assigned to 5.14%, 5.64%, 6.87%, 7.60%, and 10.51 % by the KO500 method, which is an HPLC method, as hemoglobin-containing samples, a proportion [HbA1c (%)] of HbAlc concentration to total hemoglobin concentration in each sample was determined by the following procedure.

(1) Preparation of calibration curve for determination of total hemoglobin concentration

Using hemoglobin B-test Wako, which is a kit for measuring a total hemoglobin (SLS-hemoglobin method), (manufactured by Wako Pure Chemical Industries, Ltd.) and a standard product (total hemoglobin concentration: 15.3 mg/mL) attached to the hemoglobin B-test Wako, measurement was carried out according to the following procedure, and an absorbance for the standard product was measured.

The standard product (10 μL) and the hemoglobin B-test Wako (250 μL) were added to a reaction cuvette to carry out a reaction at 37°C for 10 minutes. An absorbance (E) of the reaction solution was measured at a main wavelength of 546 nm and a sub-wavelength of 660 nm to afford an absorbance for the standard product. Measurement was carried out by the same method except that a physiological saline was used in place of the standard product to afford an absorbance for the physiological saline. The absorbance for the physiological saline was subtracted from the absorbance for the standard product to afford a blank-corrected absorbance for the standard product. Based on the blank-corrected absorbance for the standard product and a blank-corrected absorbance (0 Abs) for the physiological saline, a calibration curve showing a relationship between a total hemoglobin concentration (μmol/L) and an absorbance was prepared.

(2) Preparation of calibration curve for determination of HbA1c concentration

For two blood cell fractions in which HbAlc concentrations were determined to be 3.64 μmol/L and 9.18 μmol/L by the KO500 method, an absorbance for each blood cell fraction was measured using the kit for measuring HbA1c of Example 1 according to the following procedure.

Each of the above-mentioned blood cell fractions (10 μL) and the first reagent (100 μL) of the kit of Example 1 were added to a reaction cuvette, a reaction was carried out at 37°C for 5 minutes (first reaction), and an absorbance (E1) of the reaction solution was measured at a main wavelength of 660 nm and a sub-wavelength of 800 nm. Subsequently, the second reagent (40 μL) of the kit of Example 1 was added to the reaction solution, a further reaction was carried out at 37°C for 5 minutes (second reaction), and an absorbance (E2) of the reaction solution was measured at a main wavelength of 660 nm and a sub-wavelength of 800 nm. E1 was subtracted from E2 to calculate an absorbance difference $\Delta E'_{1A}$, and this was taken as an absorbance for each blood cell fraction. An absorbance difference $\Delta E'_{\text{physiological saline}}$ was calculated by the same method except that a physiological saline was used in place of each of the above-mentioned blood cell fractions, and this was taken as an absorbance for the physiological saline. An absorbance for the physiological saline was measured using a physiological saline in place of the blood cell fraction. The absorbance for the physiological saline was subtracted from the absorbance for each of the blood cell fractions to afford a blank-corrected absorbance for the blood cell fraction. Based on the blank-corrected absorbance for the blood cell fraction and a blank-corrected absorbance (0 Abs) for the physiological saline, a calibration curve showing a relationship between an HbAlc concentration (μmol/L) and an absorbance was prepared.

(3) Determination of total hemoglobin concentration in each blood cell fraction

For each of the 5 concentrations of whole blood samples, centrifugation was carried out at 2,200 rpm (800 × G) at 25°C for 5 minutes to obtain a blood cell fraction. For each blood cell fraction, measurement was carried out by the same method as in (1) by using hemoglobin B-test Wako, and a total hemoglobin concentration (μmol/L) in each blood cell fraction was determined based on the obtained measured value and the calibration curve of (1).

(4) Determination of HbAlc concentration in each blood cell fraction

For each blood cell fraction obtained in (3), measurement was carried out by the same method as in (2) using the kit of Example 1 of the present invention, and an HbAlc concentration (μmol/L) in each blood cell fraction was determined from the measured value thus obtained and the calibration curve of (2).

(5) Determination of HbAlc (%) (proportion of HbAlc concentration to total hemoglobin concentration)

Based on the total hemoglobin concentration (μmol/L) in each blood cell fraction determined in the above (3) and the HbAlc concentration (μmol/L) in each blood cell fraction determined in the above (4), HbAlc (%) was calculated as an NGSP value (international standard value) by the following Expression (II).

$$\text{HBA1c}(\%) = [\text{HBA1c concentration } (\mu\text{mol/L})]/[\text{total hemoglobin concentration } (\mu\text{mol/L})] \times 98.2 + 1.97 \quad \text{(II)}$$

(6) Determination of HbAlc (%) in same blood cell fraction by KO500 method

Using the same blood cell fraction as the blood cell fraction used for determining HbAlc (%) in the above (5), HbAlc (%) in each blood cell fraction was determined by the KO500 method using HPLC.

(7) Correlation between measuring method of present invention and KO500 method

Based on the HbAlc (%) determined in the above (5) using the measuring method of the present invention and the HbAlc (%) determined in the above (6) using the KO500 method, a correlation between the measuring method of the present invention and the KO500 method was verified to afford a correlation coefficient. As a result, the correlation coefficient between both measurement methods was 1.000 to afford a good correlation between both measurement methods. Accordingly, it proved that HbAlc in a sample can be accurately measured by the measuring method of the present invention using the kit of Example 1.

[Example 6]

HbAlc (%) in each blood cell fraction was determined by the same procedure as in Example 5 except that the kit of Example 2 was used in place of the kit of Example 1, and a correlation between the measuring method using the kit of Example 2 and the KO500 method was verified and a correlation coefficient was determined. As a result, the correlation coefficient between both measurement methods was 0.999 to afford a good correlation between both measurement methods. Accordingly, it proved that HbAlc in a sample can be accurately measured by the measuring method of the present invention using the kit of Example 2.

[Example 7]

HbAlc (%) in each blood cell fraction was determined by the same procedure as in Example 5 except that the kit of Example 3 was used in place of the kit of Example 1, and a correlation between the measuring method using the kit of Example 3 and the KO500 method was verified and a correlation coefficient was determined. As a result, the correlation coefficient between both measurement methods was 0.999 to afford a good correlation between both measurement methods. Accordingly, it proved that even in the measuring method of the present invention using the kit of Example 3 that does not contain catalase in the first reagent, HbAlc in a sample can be accurately measured by eliminating hydrogen peroxide generated by a reaction of glycated amino acid and/or glycated peptide in whole blood with FPOX-CET by a reducing substance such as hemoglobin or ascorbic acid in the whole blood.

[Example 8]

HbAlc (%) in each blood cell fraction was determined by the same procedure as in Example 5 except that the kit of Example 4 was used in place of the kit of Example 1, and a correlation between the measuring method using the kit of Example 4 and the KO500 method was verified and a correlation coefficient was determined. As a result, the correlation coefficient between both measurement methods was 0.999 to afford a good correlation between both measurement methods. Accordingly, it proved that even in the measuring method of the present invention using the kit of Example 4 that does not contain catalase in the first reagent, HbA1c in a sample can be accurately measured by eliminating hydrogen peroxide generated by a reaction of glycated amino acid and/or glycated peptide in whole blood with FPOX-CET by a reducing substance such as hemoglobin or ascorbic acid in the whole blood.

[Comparative Example 1]

Kits for measuring HbA1c comprising the following first reagent and second reagent were prepared. This kit is the same as the kit of Example 1 except that FPOX-CET is not contained in the first reagent.

| First reagent | |
|---|---|
| Bis-Tris (pH 7.0) | 50 mmol/L |
| NIKKOL LMT | 1.5 g/L |
| DA-67 | 40 μmol/L |
| Catalase | 100 kU/L |

| Second reagent | |
|---|---|
| Bis-Tris (pH 7.0) | 50 mmol/L |
| FPOX-47Δ3 | 1.5 kU/L |
| Peroxidase | 200 kU/L |
| Sodium azide | 0.1 g/L |

[Comparative Example 2]

Kits for measuring HbA1c comprising the following first reagent and second reagent were prepared. This kit is the same as the kit of Example 2 except that FPOX-CET is not contained in the first reagent.

First reagent

| | |
|---|---|
| Bis-Tris (pH 7.0) | 50 mmol/L |
| NIKKOL LMT | 1.5 g/L |
| Peroxidase | 40 kU/L |
| Catalase | 100 kU/L |

Second reagent

| | |
|---|---|
| Bis-Tris (pH 7.0) | 50 mmol/L |
| FPOX-47Δ3 | 1.5 kU/L |
| DA-67 | 120 μmol/L |
| Sodium azide | 0.1 g/L |

[Comparative Example 3]

Kits for measuring HbA1c comprising the following first reagent and second reagent were prepared. This kit is the same as the kit of Example 3 except that FPOX-CET is not contained in the first reagent.

First reagent

| | |
|---|---|
| Bis-Tris (pH 7.0) | 50 mmol/L |
| NIKKOL LMT | 1.5 g/L |
| DA-67 | 40 μmol/L |

Second reagent

| | |
|---|---|
| Bis-Tris (pH 7.0) | 50 mmol/L |
| FPOX-47Δ3 | 1.5 kU/L |
| Peroxidase | 200 kU/L |
| Sodium azide | 0.1 g/L |

[Comparative Example 4]

Kits for measuring HbA1c comprising the following first reagent and second reagent were prepared. This kit is the same as the kit of Example 4 except that FPOX-CET is not contained in the first reagent.

First reagent

| | |
|---|---|
| Bis-Tris (pH 7.0) | 50 mmol/L |
| NIKKOL LMT | 1.5 g/L |
| Peroxidase | 40 kU/L |

Second reagent

| | |
|---|---|
| Bis-Tris (pH 7.0) | 50 mmol/L |
| FPOX-47Δ3 | 1.5 kU/L |
| DA-67 | 120 μmol/L |
| Sodium azide | 0.1 g/L |

[Experimental Example 1]

Using whole blood having 4 μmol/L of fructosyl valine, which was prepared by adding fructosyl valine to the whole blood used in Example 5, in which the HbAlc concentration was value-assigned to 5.14% by the KO500 method, which is an HPLC method, as a hemoglobin-containing sample, and using each kit of Examples 1 to 4 and Comparative Examples 1 to 4 as the kits, an HbA1c concentration (%) [hereinafter referred to as HbAlc concentration A (%)] in each hemoglobin-containing sample was calculated by the same method as in Example 5. Similarly, using whole blood used in Example 5 in which the HbA1c concentration was value-assigned to 5.14% by the KO500 method, which is an HPLC method, as a hemoglobin-containing sample, and using each kit of Examples 1 to 4 and Comparative Examples 1 to 4 as the kits, an HbA1c concentration (%) [hereinafter referred to as HbA1c concentration B (%)] in each hemoglobin-containing sample was calculated by the same method as in Example 5. Table 1 shows values obtained by subtracting the HbAlc concentration B (%) from the HbA1c concentration A (%) [hereinafter referred to as ΔHbA1c concentration (%)]. In Table 1, Enzyme 1 indicates an enzyme that catalyzes a reaction of oxidizing glycated amino acid or glycated peptide to generate hydrogen peroxide without oxidizing glycated hemoglobin and Enzyme 2 indicates an enzyme that catalyzes a reaction of oxidizing glycated hemoglobin to generate hydrogen peroxide.

[Table 1]

| Kit | First reagent | | | | Second reagent | | | ΔHbA1c concentration (%) |
|---|---|---|---|---|---|---|---|---|
| | Enzyme 1 | DA-67 | POD | Catalase | Enzyme 2 | DA-67 | POD | |
| Example 1 | FPOX-CET | + | - | + | FPOX-47Δ3 | + | + | -0.1 |
| Example 2 | FPOX-CET | - | + | + | FPOX-47Δ3 | + | - | -0.2 |
| Example 3 | FPOX-CET | + | - | - | FPOX-47Δ3 | - | + | -0.2 |
| Example 4 | FPOX-CET | - | + | - | FPOX-47Δ3 | + | - | -0.1 |
| Comparative Example 1 | - | + | - | + | FPOX-47Δ3 | - | + | 2.0 |
| Comparative Example 2 | - | - | + | + | FPOX-47Δ3 | + | - | 1.9 |
| Comparative Example 3 | - | + | - | - | FPOX-47Δ3 | - | + | 2.0 |
| Comparative Example 4 | - | - | + | - | FPOX-47Δ3 | + | - | 1.8 |

As is apparent from Table 1, in a case where kits of Comparative Examples 1 to 4 in which FPOX-CET, which is an enzyme that catalyzes a reaction of oxidizing glycated amino acid or glycated peptide to generate hydrogen peroxide without oxidizing glycated hemoglobin, was not contained in the first reagent were used, fructosyl valine in the hemoglobin-containing sample reacted with FPOX-47Δ3 in the second reagent, and coloration caused by the generated hydrogen peroxide was observed. As a result, coloration caused by the fructosyl valine was observed. On the other hand, in a case where kits of Examples 1 to 4 in which FPOX-CET, which is an enzyme that catalyzes a reaction of oxidizing glycated amino acid or glycated peptide to generate hydrogen peroxide without oxidizing glycated hemoglobin, was contained were used, fructosyl valine in the hemoglobin-containing sample reacted with the FPOX-CET in the first reagent, and the generated hydrogen peroxide was eliminated by catalase and/or reducing substances such as hemoglobin or ascorbic acid in whole blood. As a result, coloration caused by fructosyl valine was not observed.

Accordingly, it proved that glycated hemoglobin in a hemoglobin-containing sample can be accurately measured without being affected by glycated amino acid or glycated peptide in a hemoglobin-containing sample by using the kit for measuring glycated hemoglobin of the present invention comprising a first reagent which comprises an enzyme that catalyzes a reaction of oxidizing glycated amino acid or glycated peptide to generate hydrogen peroxide without oxidizing glycated hemoglobin, catalase, and a leuco-type chromogen and a second reagent which comprises a peroxidase and an enzyme that catalyzes a reaction of oxidizing glycated hemoglobin to generate hydrogen peroxide.

In addition, it proved that glycated hemoglobin in a hemoglobin-containing sample can be accurately measured without being affected by glycated amino acid or glycated peptide in a hemoglobin-containing sample by using the kit for measuring glycated hemoglobin of the present invention comprising a first reagent which comprises an enzyme that catalyzes a reaction of oxidizing glycated amino acid or glycated peptide to generate hydrogen peroxide without oxidizing glycated hemoglobin, catalase, and a peroxidase and a second reagent which comprises a leuco-type chromogen and an enzyme that catalyzes a reaction of oxidizing glycated hemoglobin to generate hydrogen peroxide.

In addition, it proved that glycated hemoglobin in a hemoglobin-containing sample can be accurately measured without being affected by glycated amino acid or glycated peptide in a hemoglobin-containing sample by using the kit for measuring glycated hemoglobin of the present invention comprising a first reagent which comprises leuco-type chromogen and an enzyme that catalyzes a reaction of oxidizing glycated amino acid or glycated peptide to generate hydrogen peroxide without oxidizing glycated hemoglobin and a second reagent which comprises a peroxidase and an enzyme that catalyzes a reaction of oxidizing glycated hemoglobin to generate hydrogen peroxide.

Furthermore, it proved that glycated hemoglobin in a hemoglobin-containing sample can be accurately measured without being affected by glycated amino acid or glycated peptide in a hemoglobin-containing sample by using the kit for measuring glycated hemoglobin of the present invention comprising a first reagent which comprises a peroxidase and an enzyme that catalyzes a reaction of oxidizing glycated amino acid or glycated peptide to generate hydrogen peroxide without oxidizing glycated hemoglobin and a second reagent which comprises a leuco-type chromogen and an enzyme that catalyzes a reaction of oxidizing glycated hemoglobin to generate hydrogen peroxide.

[Reference Example 1] Construction of FPOX-47Δ3

(1) Construction of FPOX-47 expression vector pTrc-FPOX-47

PCR was carried out using an expression plasmid pTrc-FPOX-42 containing a DNA consisting of a base sequence represented by SEQ ID NO: 1 as a template DNA, FPOX-42 F342V-F consisting of a base sequence represented by SEQ ID NO: 2 and FPOX-42 F342V-R consisting of a base sequence represented by SEQ ID NO: 3 as a forward primer and a reverse primer, respectively, with the following reagent composition and under the following PCR conditions to obtain a PCR product. pTrc-FPOX-42 is an expression plasmid containing DNA encoding FPOX-42, which is an enzyme that catalyzes a reaction of oxidizing glycated hemoglobin to generate hydrogen peroxide, and can be constructed according to a method disclosed in PCT International Publication No. WO2015/005258. PCR was carried out based on a protocol of DNA polymerase "KOD-Plus-", which is a PCR kit manufactured by Toyobo Co., Ltd.

(Reagent composition)

| | |
|---|---|
| • KOD-Plus-buffer | |
| • Template DNA (pTrc-FPOX-42) | 1 to 2 ng/μL |
| • Forward primer (FPOX-42 F342V-F) | 0.3 μmol/L |
| • Reverse primer (FPOX-42 F342V-R) | 0.3 μmol/L |
| • dNTP mixed solution | 0.2 mmol/Leach |
| • MgSO4 1 mmol/L | |
| • DNA polymerase (KOD-Plus-) | 0.02 U/μL |
| • Sterile water was added to make | 50 μL. |

(PCR conditions)

1. 94°C for 2 minutes

2. 98°C for 15 seconds

3. 60°C for 30 seconds

4. 4. 68°C for 6 minutes

5. Repeat of 2 to 4 (all 30 cycles)

6. 68°C for 10 minutes

1 μL of "restriction enzyme Dpn I" manufactured by New England Biolabs, Inc. was added to 50 μL of the

obtained PCR product, and incubation was carried out at 37°C for 1 hour to decompose the template DNA. The PCR product which had been subjected to the restriction enzyme treatment was purified using "Wizard SV Gel and PCR Clean-Up System" manufactured by Promega according to a protocol of the kit to obtain a purified sample of the PCR product.

Subsequently, the purified sample of the obtained PCR product was used to transform an E. coli competent cell "Competent high DH 5α" manufactured by Toyobo Co., Ltd. Colonies grown on an LB agar medium containing 50 mg/L ampicillin were selected and plasmids were extracted using "Wizard Plus SV Minipreps DNA Purification" manufactured by Promega according to a protocol of the kit.

By performing sequence analysis of the extracted plasmids with a DNA sequencer, it was identified whether a plasmid pTrc-FPOX-47 that contains DNA having a base sequence represented by SEQ ID NO: 4, which encodes FPOX-47, was constructed. For the sequence analysis, pTrc-F consisting of a base sequence represented by SEQ ID NO: 5 and PTrc-R consisting of a base sequence represented by SEQ ID NO: 6, which reflect base sequences immediately before and after a multiple cloning site of a pTrc99a vector (manufactured by GE Healthcare Bioscience), were used as a forward primer and a reverse primer, respectively.

(2) Construction of FPOX-47Δ3-expressing vector pTrc-FPOX-47Δ3

FPOX-47Δ3-R consisting of a base sequence represented by SEQ ID NO: 7 which had been obtained by removing 9 bases positioned immediately before a termination codon at a 3' end of a base sequence encoding FPOX-47 contained in the plasmid pTrc-FPOX-47 and adding a termination codon and a Bam HI restriction enzyme recognition site, and pTrc-F consisting of a base sequence represented by SEQ ID NO: 5, which reflects a base sequence immediately before the multiple cloning site of pTrc99a vector, were designed and chemically synthesized.

Using pTrc-F and FPOX-47Δ3-R as a primer, PCR was carried out using pTrc-FPOX-47 as a template with the following reagent composition and under the following PCR conditions to obtain a PCR product which contains a DNA fragment containing a base sequence that encodes deletion variant, FPOX-47Δ3 in which three amino acids at a C terminus of FPOX-47 are deleted. PCR was carried out based on a protocol of DNA polymerase "KOD-Plus-", which is a PCR kit manufactured by Toyobo Co., Ltd.

(Reagent composition)

| | |
|---|---|
| • KOD-Plus-buffer | |
| • Template DNA (pTrc-FPOX-47) | 0.2 to 0.5 ng/μL |
| • Forward primer (pTrc-F) | 0.3 μmol/L |
| • Reverse primer (FPOX-47Δ3-R) | 0.3 μmol/L |
| • dNTP mixed solution | 0.2 mmol/Leach |
| • MgSO4 | 1mmol/L |
| • DNA polymerase (KOD-Plus-) | 0.02 U/μL |
| • Sterile water was added to make | 50 μL. |

(PCR conditions)

1. 94°C for 2 minutes
2. 98°C for 15 seconds
3. 60°C for 30 seconds
4. 68°C for 2 minutes
5. Repeat of 2 to 4 (all 30 cycles)
6. 68°C for 5 minutes

Using a portion of the obtained PCR product, electrophoresis was carried out on a 1% agarose TAE gel to identify whether a DNA fragment of an assumed size was obtained.

The obtained PCR product was purified using "Wizard SV Gel and PCR Clean-Up system" manufactured by Promega according to a protocol of the kit to obtain a DNA fragment that contains DNA encoding FPOX-47Δ3. The DNA fragment thus obtained was treated with two types of restriction enzymes, Nco I and Bam HI (both manufactured by Takara Bio Inc.), at 37°C for 1 hour, and the obtained reaction mixture was purified using "Wizard SV Gel and PCR Clean-Up System" manufactured by Promega according to a protocol of the kit to obtain a restriction enzyme-treated fragment.

In addition, pTrc-FPOX-15 expressing a FPOX-15 having no activity of oxidizing fructosyl hexapeptide, which is described in PCT International Publication No. WO2010/041715, was similarly treated with the same restriction enzymes, electrophoresis was carried out on a 1% agarose TAE gel, a DNA fragment corresponding to the vector was excised, and the DNA fragment was purified using Wizard SV Gel and PCR Clean-Up system to obtain a plasmid fragment from which DNA encoding FPOX-15 was removed.

The obtained restriction enzyme-treated fragment was mixed with the plasmid fragment from which DNA encoding FPOX-15 was removed, ligation was carried out using a DNA Ligation Kit (manufactured by Takara Bio Inc.), and then the ligation product was introduced into an E. coli competent cell "Competent high DH5α" manufactured by Toyobo Co., Ltd.

The E. coli into which the ligation product had been introduced was inoculated on an LB agar medium containing 50 mg/L ampicillin, cultured at 37°C for 14 hours, and emerging colonies were selected. The selected colonies were cultured in an LB liquid medium containing 50 mg/L ampicillin at 37°C for 14 hours and plasmids were prepared using "Wizard Plus SV Minipreps DNA Purification" manufactured by Promega according to a protocol of the kit. PCR was carried out using pTrc-F consisting of a base sequence represented by SEQ ID NO: 5 and pTrc-R consisting of a base sequence represented by SEQ ID NO: 6 as a forward primer and a reverse primer, respectively, to identify a base sequence of each of the prepared plasmids and identify whether a base sequence represented by SEQ ID NO: 8 in which nine bases on a 3' side except a termination codon in the base sequence of FPOX-47 were deleted was contained on the plasmid. This plasmid is called pTrc-FPOX-47Δ3.

(3) Acquisition of FPOX-47Δ3

E. coli containing pTrc-FPOX-47Δ3 obtained in the above (2) was shake-cultured in an LB medium containing 50 mg/L ampicillin at 37°C for 14 hours. Purification of the obtained culture solution was carried out according to the purification method of glycated peptide oxidases FPOX-9 and FPOX-15 described in PCT International Publication No. WO 2010/041715 to obtain a solution of purified FPOX-47Δ3. An amino acid sequence of FPOX-47 and an amino acid sequence of FPOX-47Δ3 are shown in SEQ ID NO: 9 and SEQ ID NO: 10, respectively.

(4) Identification of enzyme activity (hereinafter referred to as glycated hemoglobin oxidase activity) that catalyzes a reaction of oxidizing glycated hemoglobin of FPOX-47Δ3 to generate hydrogen peroxide

A glycated hemoglobin oxidase activity of FPOX-47Δ3 toward HbAlc was identified by using the solution of purified FPOX-47Δ3 obtained in the above (3) with the following measuring kit and measuring procedure.

<Measuring kit A>

First reagent

| | |
|---|---|
| Bis-Tris (pH 7.0) | 50 mmol/L |
| DA-67 | 40 μmol/L |
| NIKKOL LMT | 1 g/L |

Second reagent

| | |
|---|---|
| Phosphate buffer solution (pH 6.5) | 10 mmol/L |
| FPOX-47Δ3 | 1.5 kU/L |
| Peroxidase | 200kU/L |

<Measuring kit a>

| First reagent | |
|---|---|
| Bis-Tris (pH 6.5) | 50 mmol/L |
| DA-67 | 40 μmol/L |

| Second reagent | |
|---|---|
| Phosphate buffer solution (pH 6.5) | 10 mmol/L |
| FPOX-47Δ3 | 1.5 kU/L |
| Peroxidase | 200 kU/L |

The measuring kit a is the same as the measuring kit A except that NIKKOL LMT is not contained in the first reagent.

<Sample>

Hemolyzed samples prepared by dilution of blood cells obtained by centrifugation of human blood with deionized water to hemolyze, and with the HbAlc concentrations of 2.6 μmol/L, 4.5 μmol/L, 6.4 μmol/L, 8.4 μmol/L determined from both of the KO500 method, which is a standard method for measuring HbA1c, and the SLS-hemoglobin method, which is one of the methods for measuring a total hemoglobin, were used as samples.

<Measuring procedure>

Each of the above-mentioned samples (10 μL) and the first reagent (100 μL) of the above-mentioned measuring kit A were added to a reaction cuvette, a reaction was carried out at 37°C for 5 minutes (first reaction), and an absorbance (E1) of the reaction solution was measured at a main wavelength of 660 nm and a sub-wavelength of 800 nm. Subsequently, the second reagent (40 μL) of the above-mentioned measuring kit A was added to the reaction solution, a further reaction was carried out at 37°C for 5 minutes (second reaction), and an absorbance of the reaction solution (E2) was measured at a main wavelength of 660 nm and a sub-wavelength of 800 nm. E1 was subtracted from E2 to calculate an absorbance difference $\Delta E'_A$.

Each of the above-mentioned samples (10 μL) and the first reagent (100 μL) of the above-mentioned measuring kit a were added to a reaction cuvette, a reaction was carried out at 37°C for 5 minutes (first reaction), and an absorbance (E3) of the reaction solution was measured at a main wavelength of 660 nm and a sub-wavelength of 800 nm. Subsequently, the second reagent (40 μL) of the above-mentioned measuring kit a was added to the reaction solution, a further reaction was carried out at 37°C for 5 minutes (second reaction), and an absorbance of the reaction solution (E4) was measured at a main wavelength of 660 nm and a sub-wavelength of 800 nm. E3 was subtracted from E4 to calculate an absorbance difference $\Delta E'_a$.

$\Delta E'_a$ was subtracted from $\Delta E'_A$, and this was taken as an absorbance difference ΔE for each sample. A relationship between an HbAlc concentration and ΔE in each sample is shown in FIG. 1.

As is apparent from FIG. 1, in the measurement using a kit (kit A) of the present invention containing N-acyltaurine, which is an anionic surfactant, a quantitative relationship was recognized between an HbAlc concentration and an absorbance. Accordingly, it proved that FPOX-47Δ3 is an enzyme having activity that catalyzes a reaction of oxidizing glycated hemoglobin to generate hydrogen peroxide.

Industrial Applicability

According to the present invention, a method and a kit are provided for measuring glycated hemoglobin in a hemoglobin-containing sample useful for diagnosis of diabetes and the like.

## Claims

**1.** A method for measuring glycated hemoglobin in a hemoglobin-containing sample, comprising:

adding an enzyme that catalyzes a reaction of oxidizing glycated amino acid or glycated peptide to generate hydrogen peroxide without oxidizing the glycated hemoglobin, to the hemoglobin-containing sample to generate hydrogen peroxide;
eliminating the generated hydrogen peroxide;
adding an enzyme that catalyzes a reaction of oxidizing glycated hemoglobin to generate hydrogen peroxide thereto to generate hydrogen peroxide; and
measuring the generated hydrogen peroxide.

**2.** The method according to Claim 1, comprising:

(1) a step of reacting an enzyme that catalyzes a reaction of oxidizing glycated amino acid or glycated peptide to generate hydrogen peroxide without oxidizing glycated hemoglobin with glycated amino acid or glycated peptide in the hemoglobin-containing sample to generate hydrogen peroxide, and eliminating the generated hydrogen peroxide;
(2) a step of adding an enzyme that catalyzes a reaction of oxidizing glycated hemoglobin to generate hydrogen peroxide to a reaction solution in step (1), and reacting the enzyme that catalyzes a reaction of oxidizing glycated hemoglobin to generate hydrogen peroxide with glycated hemoglobin to generate hydrogen peroxide;
(3) a step of measuring the hydrogen peroxide generated in step (2); and
(4) a step of determining a concentration of the glycated hemoglobin in the hemoglobin-containing sample by collating the measured value of step (3) with a calibration curve showing a relationship between a concentration of glycated hemoglobin and a measured value of hydrogen peroxide, prepared in advance by performing the same steps as above (1) to (3), using glycated hemoglobin with known concentrations instead of the hemoglobin-containing sample.

**3.** The method according to Claim 1 or 2, wherein the elimination of the hydrogen peroxide is carried out by catalase.

**4.** The method according to Claim 3, wherein the measurement of the hydrogen peroxide is carried out in the presence of a catalase inhibitor.

**5.** The method according to Claim 4, wherein the catalase inhibitor is azide.

**6.** The method according to Claim 1 or 2, wherein the elimination of the hydrogen peroxide is carried out by a combination of a peroxidase and one of two oxidative coupling-type chromogens used in a chromogenic oxidative coupling reaction.

**7.** The method according to any one of Claims 1 to 6, wherein the elimination of the hydrogen peroxide is carried out by a reducing substance in the sample.

**8.** The method according to any one of Claims 1 to 7, wherein the measurement of the hydrogen peroxide is carried out with a peroxidase and a leuco-type chromogen.

**9.** The method according to Claim 8, wherein the leuco-type chromogen is a phenothiazine-based chromogen.

**10.** The method according to Claim 9, wherein the phenothiazine-based chromogen is 10-(carboxymethylaminocarbonyl)-3,7-bis(dimethylamino)phenothiazine or a salt thereof.

**11.** A kit for measuring glycated hemoglobin in a hemoglobin-containing sample, comprising:

a first reagent which comprises catalase and an enzyme that catalyzes a reaction of oxidizing glycated amino acid or glycated peptide to generate hydrogen peroxide without oxidizing glycated hemoglobin; and
a second reagent which comprises a catalase inhibitor and an enzyme that catalyzes a reaction of oxidizing glycated hemoglobin to generate hydrogen peroxide.

**12.** The kit according to Claim 11, wherein the catalase inhibitor is azide.

**13.** The kit according to Claim 11 or 12, wherein a peroxidase and a leuco-type chromogen are further contained in the first reagent and the second reagent, respectively, or in the second reagent and the first reagent, respectively.

**14.** A kit for measuring glycated hemoglobin in a hemoglobin-containing sample, comprising:

a first reagent which comprises an enzyme that catalyzes a reaction of oxidizing glycated amino acid or glycated peptide to generate hydrogen peroxide without oxidizing glycated hemoglobin and a combination of a peroxidase and one of two oxidative coupling-type chromogens used in a chromogenic oxidative coupling reaction; and
a second reagent which comprises a leuco-type chromogen and an enzyme that catalyzes a reaction of oxidizing glycated hemoglobin to generate hydrogen peroxide.

**15.** A kit for measuring glycated hemoglobin in a hemoglobin-containing sample, comprising:

a first reagent which comprises an enzyme that catalyzes a reaction of oxidizing glycated amino acid or glycated peptide to generate hydrogen peroxide without oxidizing glycated hemoglobin; and

a second reagent which comprises an enzyme that catalyzes a reaction of oxidizing glycated hemoglobin to generate hydrogen peroxide,

wherein a peroxidase and a leuco-type chromogen are contained in the first reagent and the second reagent, respectively, or in the second reagent and the first reagent, respectively.

**16.** The kit according to any one of Claims 13 to 15,
wherein the leuco-type chromogen is a phenothiazine-based chromogen.

**17.** The kit according to Claim 16,
wherein the phenothiazine-based chromogen is 10-(carboxymethylaminocarbonyl)-3,7-bis(dimethylamino)phenothiazine or a salt thereof.

FIG. 1

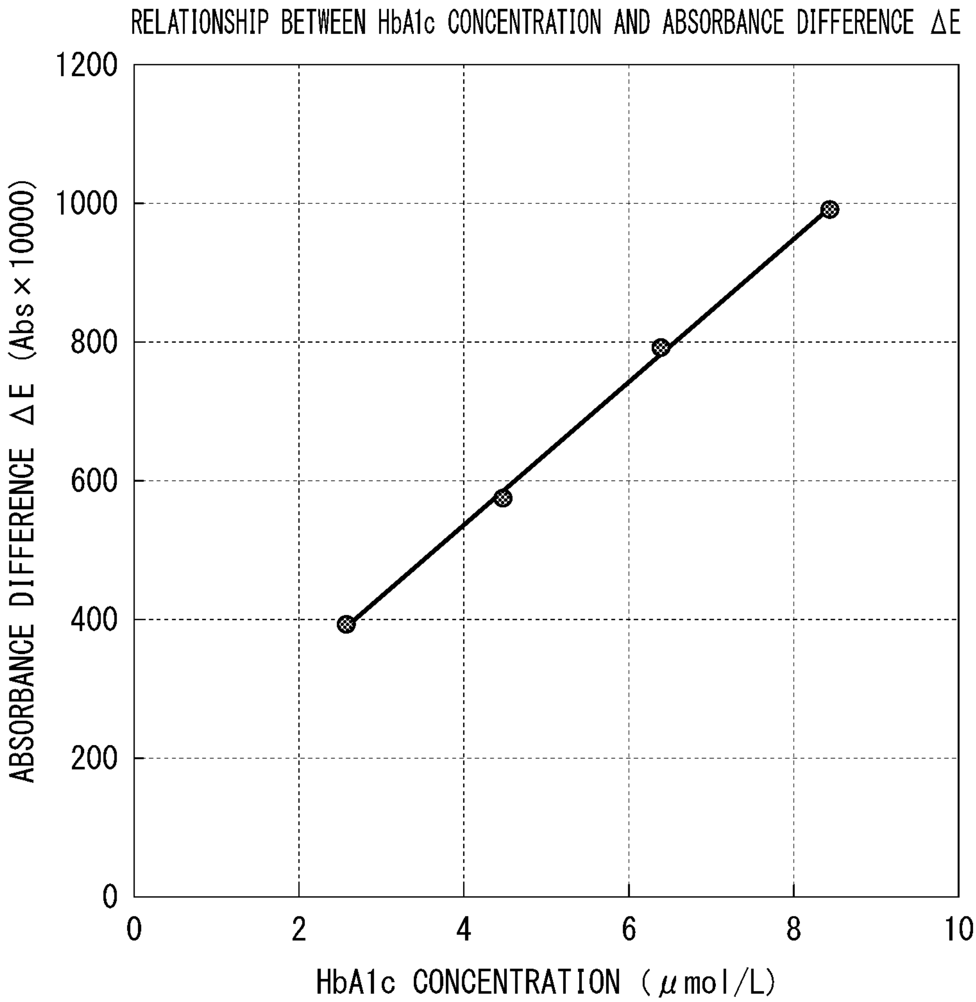
RELATIONSHIP BETWEEN HbA1c CONCENTRATION AND ABSORBANCE DIFFERENCE ΔE
1200
1000
800
600
400
200
0
ABSORBANCE DIFFERENCE ΔE (Abs×10000)
0
2
4
6
8
10
HbA1c CONCENTRATION (μmol/L)

## INTERNATIONAL SEARCH REPORT

International application No. PCT/JP2018/020319

A. CLASSIFICATION OF SUBJECT MATTER
Int.Cl. C12Q1/26(2006.01)i, C12M1/34(2006.01)i, C12Q1/28(2006.01)i, C12Q1/30(2006.01)i, G01N33/72(2006.01)i

According to International Patent Classification (IPC) or to both national classification and IPC

B. FIELDS SEARCHED

Minimum documentation searched (classification system followed by classification symbols)
Int.Cl. C12Q1/26, C12M1/34, C12Q1/28, C12Q1/30, G01N33/72

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/MEDLINE/EMBASE/BIOSIS (STN), UniProt/GeneSeq

C. DOCUMENTS CONSIDERED TO BE RELEVANT

| Category* | Citation of document, with indication, where appropriate, of the relevant passages | Relevant to claim No. |
|---|---|---|
| Y | JP 2010-011876 A (ARKRAY, INC.) 21 January 2010, claims, paragraphs [0014]-[0016], [0217], examples & US 2005/0042709 A1, claims, paragraphs [0014]-[0016], [0243], examples & EP 1477569 A1 & CN 1625601 A | 1-17 |
| Y | WO 2015/005258 A1 (KYOWA MEDEX CO., LTD.) 15 January 2015, claims, paragraphs [0143]-[0160], examples & US 2016/0138073 A1, claims, paragraphs [0277]-[0298], examples & EP 3020810 A4 & CN 105431533 A | 1-17 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 August 2018 (15.08.2018) | 28 August 2018 (28.08.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2018/020319

C (Continuation). DOCUMENTS CONSIDERED TO BE RELEVANT

| Category* | Citation of document, with indication, where appropriate, of the relevant passages | Relevant to claim No. |
|---|---|---|
| Y | 小川徳之 ほか, 構造情報に基づく部位特異的変異及びランダム変異導入に依るヘモグロビンＡ１ｃダイレクトオキシダーゼの造成, 日本農芸化学会 2016 年度大会講演要旨集（オンライン）, 05 March 2016, 3J002, purpose, methods and results, non-official translation (OGAWA, Noriyuki et al., "Compositions of hemoglobin A1C direct oxidase by introduction of site-specific mutant and random mutant based on structural information", Lecture abstracts (online) of the 2016 conference of the Japan Society for Bioscience, Biotechnology, and Agrochemistry) | 1-17 |
| Y | WO 2015/060429 A1 (KIKKOMAN CORPORATION) 30 April 2015, claims, examples, paragraph [0204] & US 2016/0251695 A1, claims, examples, paragraph [0546] & EP 3061819 A4 & KR 10-2016-0074610 A & CN 105683374 A | 1-17 |
| Y | JP 2007-289202 A (ARKRAY, INC.) 08 November 2007, claims, paragraphs [0014], [0016], [0050], examples & US 2004/0248226 A1, claims, paragraphs [0014], [0016], [0062], examples & EP 1443115 A1 & CN 1568371 A | 1-17 |
| Y | WO 2007/072941 A1 (KYOWA MEDEX CO., LTD.) 28 June 2007, claims, paragraphs [0085]-[0098], [0134]-[0139] (Family: none) | 1-17 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2017107002 A
- WO 2015005258 A
- WO 2015005257 A
- WO 2015060429 A
- JP 2011045389 A
- WO 2003033729 A
- WO 2010041715 A

### Non-patent literature cited in the description

- *Clin Chem Lab Med,* 1998, vol. 36, 299-308
- *Diabetes,* 1978, vol. 27 (2), 102-107
- *Japanese Journal of Clinical Laboratory Automation,* 1993, vol. 18 (4), 620
- *J. Agric. Food Chem.,* 1976, vol. 24 (1), 70-73

SEQUENCE LISTING

<110> KYOWA MEDEX CO.,LTD

<120> Method for measurement of the glycosylated hemoglobin

<130> 1000P12449WO0

<140> JP2017-107002
<141> 2017-05-30

<160> 10

<170> PatentIn version 3.5

<210> 1
<211> 1317
<212> DNA
<213> Artificial Sequence

<220>
<223> FPOX-42

<400> 1
```
atggcgcccc gagccaacac caaaatcatc gtcgtcggcg gcggcggcac aatgggctcg      60

tcgacagccc tacacctcct gcgcgccggc tacacgccgt ccaacatcac agtgctcgac     120

acgtacccta tcccttccgc acagtctgca ggctacgacc tgaacaagat cttcggcatc     180

tcaggcgcga acaagcatga cttacaactc tcgcttgagg cgttcgacat gtggaaaaat     240

gatcctctat tcaagccgtt tttccacaat gttggacaga tggacgtctc ttcaacagaa     300

gaaggcatca aacgccttcg ccgcagatac cagtctcttc tccgcgcagg cattgggctc     360

gagaagacga atttcctgct ggaaagtgaa gacgagatcc tggctaaagc gccgcatttc     420

acgcgggagc agattaaagg ctggaaaggg ctgttctgtg gcgacggcgg ttggctcgct     480

gcagccaaag ccatcaatgc catcgggcag ttcctcaagg aacagggcgt caagtttgga     540

tttggcgagg ccggcacgtt caaaaagcca ctcttcgccg atgccgacga gaagacgtgc     600

atcggcgtcg aaactgtaga cggcacaaaa tactacgccg acaaggtcgt tctagcagct     660

ggtgcctgga gttcgacgtt ggtcgatctg gaggagcagt gcgtttcaaa ggcctgggtc     720

tttgcccaca tccaactgac gcccgctgaa gcagccgcgt acaagaacac tcctgttata     780

tacgacggtg actatgggtt tttcattgag ccggacgaga acggcatcat aaaagtctgc     840

gacgaattcc ctggcttcac gcacttcaag atgcaccagc cgtacggctc accggtgccc     900

aaattgatct ctgtgcctcg ctcccatgcg aagcacccca cagatacata cccgcacgcg     960

tcggaggtca ccatcaaaaa ggctatcaac cggttcctgc cgaggttcaa tgacaaggaa    1020

ctgtttaaca gggccatgtg ctggtgcacc gataccgcgg atagcaatct gcttgtttgt    1080

gagcatccac gctggaaggg gttttatctt gcaacagggg acagcgggca ttcgttcaag    1140

ttgctgccga atattggaaa gcacgttgtc gagttattgg aggggaggct ggaaagtgtg    1200
```

tttaaggatg cttggaggtg gaggcctggc agtggggatg cattaaagag tagacgggct 1260

gcgcctgcga aggacctggc ggatatgccg gggtggagga atgaggcaaa gatgtag 1317

<210> 2
<211> 31
<212> DNA
<213> Artificial Sequence

<220>
<223> FPOX-42 F342V-F

<400> 2
gaggttcaat gacaaggaac tggtgaacag g 31

<210> 3
<211> 30
<212> DNA
<213> Artificial Sequence

<220>
<223> FPOX-42 F342V-R

<400> 3
gcaccagcac atggccctgt tcaccagttc 30

<210> 4
<211> 1317
<212> DNA
<213> Artificial Sequence

<220>
<223> FPOX-47

<400> 4
atggcgcccc gagccaacac caaaatcatc gtcgtcggcg gcggcggcac aatgggctcg 60

tcgacagccc tacacctcct gcgcgccggc tacacgccgt ccaacatcac agtgctcgac 120

acgtacccta tcccttccgc acagtctgca ggctacgacc tgaacaagat cttcggcatc 180

tcaggcgcga acaagcatga cttacaactc tcgcttgagg cgttcgacat gtggaaaaat 240

gatcctctat tcaagccgtt tttccacaat gttggacaga tggacgtctc ttcaacagaa 300

gaaggcatca aacgccttcg ccgcagatac cagtctcttc tccgcgcagg cattgggctc 360

gagaagacga atttcctgct ggaaagtgaa gacgagatcc tggctaaagc gccgcatttc 420

acgcgggagc agattaaagg ctggaaaggg ctgttctgtg gcgacggcgg ttggctcgct 480

gcagccaaag ccatcaatgc catcgggcag ttcctcaagg aacagggcgt caagtttgga 540

tttggcgagg ccggcacgtt caaaaagcca ctcttcgccg atgccgacga gaagacgtgc 600

atcggcgtcg aaactgtaga cggcacaaaa tactacgccg acaaggtcgt tctagcagct 660

ggtgcctgga gttcgacgtt ggtcgatctg gaggagcagt gcgtttcaaa ggcctgggtc 720

tttgcccaca tccaactgac gcccgctgaa gcagccgcgt acaagaacac tcctgttata 780

tacgacggtg actatgggtt tttcattgag ccggacgaga acggcatcat aaaagtctgc 840

gacgaattcc ctggcttcac gcacttcaag atgcaccagc cgtacggctc accggtgccc 900

aaattgatct ctgtgcctcg ctcccatgcg aagcacccca cagatacata cccgcacgcg 960

tcggaggtca ccatcaaaaa ggctatcaac cggttcctgc cgaggttcaa tgacaaggaa 1020

ctggtgaaca gggccatgtg ctggtgcacc gataccgcgg atagcaatct gcttgtttgt 1080

gagcatccac gctggaaggg gttttatctt gcaacagggg acagcgggca ttcgttcaag 1140

ttgctgccga atattggaaa gcacgttgtc gagttattgg aggggaggct ggaaagtgtg 1200

tttaaggatg cttggaggtg gaggcctggc agtggggatg cattaaagag tagacgggct 1260

gcgcctgcga aggacctggc ggatatgccg ggtggagga atgaggcaaa gatgtag 1317

<210> 5
<211> 21
<212> DNA
<213> Artificial Sequence

<220>
<223> pTrc-F

<400> 5
caattaatca tccggctcgt a 21

<210> 6
<211> 20
<212> DNA
<213> Artificial Sequence

<220>
<223> pTrc-R

<400> 6
cttctgagtt cggcatgggg 20

<210> 7
<211> 39
<212> DNA
<213> Artificial Sequence

<220>
<223> FPOX-47\203¢3-R

<400> 7
atgggatccc tactcattcc tccaccccgg catatccgc 39

<210> 8
<211> 1308
<212> DNA
<213> Artificial Sequence

<220>

<223> FPOX-47\203¢3

<400> 8
atggcgcccc gagccaacac caaaatcatc gtcgtcggcg gcggcggcac aatgggctcg 60

tcgacagccc tacacctcct gcgcgccggc tacacgccgt ccaacatcac agtgctcgac 120

acgtacccta tcccttccgc acagtctgca ggctacgacc tgaacaagat cttcggcatc 180

tcaggcgcga acaagcatga cttacaactc tcgcttgagg cgttcgacat gtggaaaaat 240

gatcctctat tcaagccgtt tttccacaat gttggacaga tggacgtctc ttcaacagaa 300

gaaggcatca aacgccttcg ccgcagatac cagtctcttc tccgcgcagg cattgggctc 360

gagaagacga atttcctgct ggaaagtgaa gacgagatcc tggctaaagc gccgcatttc 420

acgcgggagc agattaaagg ctggaaaggg ctgttctgtg gcgacggcgg ttggctcgct 480

gcagccaaag ccatcaatgc catcgggcag ttcctcaagg aacagggcgt caagtttgga 540

tttggcgagg ccggcacgtt caaaaagcca ctcttcgccg atgccgacga gaagacgtgc 600

atcggcgtcg aaactgtaga cggcacaaaa tactacgccg acaaggtcgt tctagcagct 660

ggtgcctgga gttcgacgtt ggtcgatctg gaggagcagt gcgtttcaaa ggcctgggtc 720

tttgcccaca tccaactgac gcccgctgaa gcagccgcgt acaagaacac tcctgttata 780

tacgacggtg actatgggtt tttcattgag ccggacgaga acggcatcat aaaagtctgc 840

gacgaattcc ctggcttcac gcacttcaag atgcaccagc cgtacggctc accggtgccc 900

aaattgatct ctgtgcctcg ctcccatgcg aagcacccca cagatacata cccgcacgcg 960

tcggaggtca ccatcaaaaa ggctatcaac cggttcctgc cgaggttcaa tgacaaggaa 1020

ctggtgaaca gggccatgtg ctggtgcacc gataccgcgg atagcaatct gcttgtttgt 1080

gagcatccac gctggaaggg gttttatctt gcaacagggg acagcgggca ttcgttcaag 1140

ttgctgccga atattggaaa gcacgttgtc gagttattgg aggggaggct ggaaagtgtg 1200

tttaaggatg cttggaggtg gaggcctggc agtggggatg cattaaagag tagacgggct 1260

gcgcctgcga aggacctggc ggatatgccg gggtggagga atgagtag 1308

<210> 9
<211> 438
<212> PRT
<213> Artificial Sequence

<220>
<223> FPOX-47

<400> 9

Met Ala Pro Arg Ala Asn Thr Lys Ile Ile Val Val Gly Gly Gly Gly
1 5 10 15

Thr Met Gly Ser Ser Thr Ala Leu His Leu Leu Arg Ala Gly Tyr Thr

20 25 30

Pro Ser Asn Ile Thr Val Leu Asp Thr Tyr Pro Ile Pro Ser Ala Gln
35 40 45

Ser Ala Gly Tyr Asp Leu Asn Lys Ile Phe Gly Ile Ser Gly Ala Asn
50 55 60

Lys His Asp Leu Gln Leu Ser Leu Glu Ala Phe Asp Met Trp Lys Asn
65 70 75 80

Asp Pro Leu Phe Lys Pro Phe Phe His Asn Val Gly Gln Met Asp Val
85 90 95

Ser Ser Thr Glu Glu Gly Ile Lys Arg Leu Arg Arg Arg Tyr Gln Ser
100 105 110

Leu Leu Arg Ala Gly Ile Gly Leu Glu Lys Thr Asn Phe Leu Leu Glu
115 120 125

Ser Glu Asp Glu Ile Leu Ala Lys Ala Pro His Phe Thr Arg Glu Gln
130 135 140

Ile Lys Gly Trp Lys Gly Leu Phe Cys Gly Asp Gly Gly Trp Leu Ala
145 150 155 160

Ala Ala Lys Ala Ile Asn Ala Ile Gly Gln Phe Leu Lys Glu Gln Gly
165 170 175

Val Lys Phe Gly Phe Gly Glu Ala Gly Thr Phe Lys Lys Pro Leu Phe
180 185 190

Ala Asp Ala Asp Glu Lys Thr Cys Ile Gly Val Glu Thr Val Asp Gly
195 200 205

Thr Lys Tyr Tyr Ala Asp Lys Val Val Leu Ala Ala Gly Ala Trp Ser
210 215 220

Ser Thr Leu Val Asp Leu Glu Glu Gln Cys Val Ser Lys Ala Trp Val
225 230 235 240

Phe Ala His Ile Gln Leu Thr Pro Ala Glu Ala Ala Ala Tyr Lys Asn
245 250 255

Thr Pro Val Ile Tyr Asp Gly Asp Tyr Gly Phe Phe Ile Glu Pro Asp
260 265 270

Glu Asn Gly Ile Ile Lys Val Cys Asp Glu Phe Pro Gly Phe Thr His
275 280 285

Phe Lys Met His Gln Pro Tyr Gly Ser Pro Val Pro Lys Leu Ile Ser
290 295 300

Val Pro Arg Ser His Ala Lys His Pro Thr Asp Thr Tyr Pro His Ala
305 310 315 320

Ser Glu Val Thr Ile Lys Lys Ala Ile Asn Arg Phe Leu Pro Arg Phe
325 330 335

Asn Asp Lys Glu Leu Val Asn Arg Ala Met Cys Trp Cys Thr Asp Thr
340 345 350

Ala Asp Ser Asn Leu Leu Val Cys Glu His Pro Arg Trp Lys Gly Phe
355 360 365

Tyr Leu Ala Thr Gly Asp Ser Gly His Ser Phe Lys Leu Leu Pro Asn
370 375 380

Ile Gly Lys His Val Val Glu Leu Leu Glu Gly Arg Leu Glu Ser Val
385 390 395 400

Phe Lys Asp Ala Trp Arg Trp Arg Pro Gly Ser Gly Asp Ala Leu Lys
405 410 415

Ser Arg Arg Ala Ala Pro Ala Lys Asp Leu Ala Asp Met Pro Gly Trp
420 425 430

Arg Asn Glu Ala Lys Met
435

<210> 10
<211> 435
<212> PRT
<213> Artificial Sequence

<220>
<223> FPOX-47\203¢3

<400> 10

Met Ala Pro Arg Ala Asn Thr Lys Ile Ile Val Val Gly Gly Gly Gly
1 5 10 15

Thr Met Gly Ser Ser Thr Ala Leu His Leu Leu Arg Ala Gly Tyr Thr
20 25 30

Pro Ser Asn Ile Thr Val Leu Asp Thr Tyr Pro Ile Pro Ser Ala Gln

```
        35                  40                  45

Ser Ala Gly Tyr Asp Leu Asn Lys Ile Phe Gly Ile Ser Gly Ala Asn
    50                  55                  60

Lys His Asp Leu Gln Leu Ser Leu Glu Ala Phe Asp Met Trp Lys Asn
65                  70                  75                  80

Asp Pro Leu Phe Lys Pro Phe Phe His Asn Val Gly Gln Met Asp Val
                85                  90                  95

Ser Ser Thr Glu Glu Gly Ile Lys Arg Leu Arg Arg Arg Tyr Gln Ser
            100                 105                 110

Leu Leu Arg Ala Gly Ile Gly Leu Glu Lys Thr Asn Phe Leu Leu Glu
        115                 120                 125

Ser Glu Asp Glu Ile Leu Ala Lys Ala Pro His Phe Thr Arg Glu Gln
    130                 135                 140

Ile Lys Gly Trp Lys Gly Leu Phe Cys Gly Asp Gly Gly Trp Leu Ala
145                 150                 155                 160

Ala Ala Lys Ala Ile Asn Ala Ile Gly Gln Phe Leu Lys Glu Gln Gly
                165                 170                 175

Val Lys Phe Gly Phe Gly Glu Ala Gly Thr Phe Lys Lys Pro Leu Phe
            180                 185                 190

Ala Asp Ala Asp Glu Lys Thr Cys Ile Gly Val Glu Thr Val Asp Gly
        195                 200                 205

Thr Lys Tyr Tyr Ala Asp Lys Val Val Leu Ala Ala Gly Ala Trp Ser
    210                 215                 220

Ser Thr Leu Val Asp Leu Glu Glu Gln Cys Val Ser Lys Ala Trp Val
225                 230                 235                 240

Phe Ala His Ile Gln Leu Thr Pro Ala Glu Ala Ala Ala Tyr Lys Asn
                245                 250                 255

Thr Pro Val Ile Tyr Asp Gly Asp Tyr Gly Phe Phe Ile Glu Pro Asp
            260                 265                 270

Glu Asn Gly Ile Ile Lys Val Cys Asp Glu Phe Pro Gly Phe Thr His
        275                 280                 285
```

```
Phe Lys Met His Gln Pro Tyr Gly Ser Pro Val Pro Lys Leu Ile Ser
    290                 295                 300

Val Pro Arg Ser His Ala Lys His Pro Thr Asp Thr Tyr Pro His Ala
305                 310                 315                 320

Ser Glu Val Thr Ile Lys Lys Ala Ile Asn Arg Phe Leu Pro Arg Phe
                325                 330                 335

Asn Asp Lys Glu Leu Val Asn Arg Ala Met Cys Trp Cys Thr Asp Thr
            340                 345                 350

Ala Asp Ser Asn Leu Leu Val Cys Glu His Pro Arg Trp Lys Gly Phe
        355                 360                 365

Tyr Leu Ala Thr Gly Asp Ser Gly His Ser Phe Lys Leu Leu Pro Asn
    370                 375                 380

Ile Gly Lys His Val Val Glu Leu Leu Glu Gly Arg Leu Glu Ser Val
385                 390                 395                 400

Phe Lys Asp Ala Trp Arg Trp Arg Pro Gly Ser Gly Asp Ala Leu Lys
                405                 410                 415

Ser Arg Arg Ala Ala Pro Ala Lys Asp Leu Ala Asp Met Pro Gly Trp
            420                 425                 430

Arg Asn Glu
        435
```